# EUROPEAN PATENT APPLICATION

(11) **EP 4 806 368 A1**
(43) Date of publication of application: **16.09.2026**
(21) Application number: 25162630.5
(22) Date of filing: 10.03.2025
(51) Int. Cl.: A61B 8/08, A61B 8/00, G06N 3/00

(54) **IMPROVED FETAL PARAMETER MEASUREMENT IN ULTRASOUND IMAGING WITH BLIND SWEEP PROTOCOL**

(71) Applicant: Koninklijke Philips N.V., 5656 AE Eindhoven (NL)
(72) Inventor: PATEL, Priyam, Eindhoven (NL); BHARAT, Shyam, Eindhoven (NL)
(74) Representative: Philips Intellectual Property & Standards

(57) **Abstract**

An apparatus includes a processor configured for communication with an ultrasound probe. The processor circuit is configured to: control the ultrasound probe to obtain a group of ultrasound images during one or more sweeps of a blind sweep protocol on a patient with a pregnancy, divide the ultrasound images into a number of sub-sweeps, and determine, based on the group of ultrasound image frames, a number of initial values of a fetal parameter and a number of initial values of a confidence for the plurality of sub-sweeps, respectively. The processor circuit is also configured to determine an initial value of the confidence that is relatively larger than other initial values, where the initial value of the confidence that is identified is specific to a sub-sweep, and output a first screen display including an instruction to a user to perform a follow-up scan with the ultrasound probe on the sub-sweep.

## Description

### FIELD OF THE INVENTION

The subject matter described herein relates to devices, systems, and methods for using ultrasound data from a blind abdominal imaging sweeps to detect fetal parameters (e.g., gestational age, fetal presentation or lie, detecting multiple pregnancies, etc.).

### BACKGROUND OF THE INVENTION

Ultrasound imaging is often used for diagnostic purposes in an office or hospital setting, but may also be used in resource-constrained care settings (e.g., homes, accident sites, ambulances, mobile health facilities, etc.) by emergency personnel, home health nurses, midwives, etc., who may lack ultrasound expertise. To facilitate ultrasound image acquisition by untrained or minimally trained users, a "blind sweep" protocol is often employed, in which the user follows pre-determined probe paths (e.g., sweeping out a pattern on the patient's abdomen) during imaging.

Ultrasound imaging is a vital component of high-quality obstetric care. For example, ultrasound detection of fetal parameters such as gestational age can allow for appropriate referral for delivery care at appropriate times. However, in rural and under-resourced communities, the scarcity of ultrasound imaging results in a considerable gap in the healthcare of pregnant mothers.

In a low resource ultrasound setting, it is often not possible to run the standard clinical workflow for fetal biometry workup, primarily due to lack of adequate clinical expertise in acquiring and interpreting the ultrasound scans, and secondly also due to lack of access to ultrasound equipment. In low-resource ultrasound settings, blind sweep scans are often used for initial fetal screening due to limited ability to perform standard clinical workflows.

Blind sweep protocols enable a novice or minimally trained ultrasound user to conduct an elementary screening for automated estimation of basic but important fetal parameters such as gestational age, fetal presentation, cardiac activity, multiple gestation, placenta location and deepest vertical pocket of amniotic fluid. Efforts are underway to integrate these capabilities into a handheld ultrasound platform (e.g., ultrasound probe connected to a tablet or phone), to enable easier uptake of the solution in low resource settings. However, these methods can produce outlier measurements of the fetal parameters, compromising accuracy. For example, due to resource constraints (e.g., memory-related performance constraints on mobile devices such as tablets or phones), these methods are optimized for minimal resource usage, and can sometimes give outlier measurements which are not correct.

The information included in this Background section of the specification, including any references cited herein and any description or discussion thereof, is included for technical reference purposes only and is not to be regarded as subject matter by which the scope of the disclosure is to be bound.

### SUMMARY OF THE INVENTION

The invention is defined by the independent claims. Advantageous embodiments are provided in the dependent claims. Disclosed is an ultrasound sweep fetal parameter detection system that, following a blind sweep protocol by an ultrasound probe on patient body with a pregnancy (sweeps at particular locations along body without trying to find specific anatomy), detects fetal parameters and prediction confidence from the captured images. The system then requests follow-up scans by the ultrasound probe for regions of interest where the prediction confidence is high, and uses the follow-up scans to make higher-confidence follow-up predictions. These higher-confidence fetal parameters can improve health outcomes for the mother and/or the baby. The ultrasound sweep fetal parameter detection system disclosed herein allows for more accurate, higher-confidence prediction of fetal parameters than are currently generated using the limited memory and processing resources available on a smartphone or tablet computer. The present disclosure makes better, more efficient use of these limited resources, in order to arrive at measurement confidences that are currently available with expert-operated, more sophisticated (e.g., dedicated, console-based) ultrasound equipment.

A system of one or more computers can be configured to perform particular operations or actions by virtue of having software, firmware, hardware, or a combination of them installed on the system that in operation causes or cause the system to perform the actions. One or more computer programs can be configured to perform particular operations or actions by virtue of including instructions that, when executed by data processing apparatus, cause the apparatus to perform the actions. One general aspect includes an apparatus with a processor circuit configured for communication with an ultrasound probe, where the processor circuit is configured to: control the ultrasound probe to obtain a first plurality of ultrasound image frames during one or more sweeps of a blind sweep protocol on a patient with a pregnancy; divide the first plurality of ultrasound image frames into a plurality of sub-sweeps; determine, based on the first plurality of ultrasound image frames, a plurality of initial values of a fetal parameter and a plurality of initial values of a confidence for the plurality of sub-sweeps, respectively; determine an initial value of the confidence that is relatively larger than other initial values of the confidence, where the initial value of the confidence that is identified is specific to a sub-sweep of the plurality of sub-sweeps; and output, to a display in communication with the processor circuit, a first screen display may include an instruction to a user to perform a follow-up scan with the ultrasound probe on the sub-sweep (i.e., the sub-sweep associated to the identified initial value of confidence). The apparatus effectively manages computational resources and reduces the likelihood of outlier measurements by breaking down large ultrasound data sets into smaller, manageable sub-sweeps, thus enabling accurate and confident fetal parameter estimations even in low-resource environments. Further, by determining initial values of both fetal parameters and their associated confidence levels for each sub-sweep, the apparatus identifies the sub-sweep with the highest confidence for follow-up scanning. This may ensure efficient use of resources by re-scanning only when necessary, thereby enhancing the accuracy and reliability of fetal measurements, even when initial confidence values are low. This may also reduce patient or user exposure to unnecessary rescans and ultrasound exposure when either no rescans are needed, or only a portion of a scan needs to be repeated. Further this may additionally reduce resource usage of the clinician or user to the minimum needed to maintain quality and enhance safety to the patient or user.

Other embodiments of this aspect include corresponding computer systems, apparatus, and computer programs recorded on one or more computer storage devices, each configured to perform the actions of the methods.

In particular, another general aspect includes a method for providing guidance to a user to perform a follow-up scan to determine a value of a fetal parameter using ultrasound data from a blind abdominal imaging sweep, comprising: controlling an ultrasound probe to obtain a first plurality of ultrasound image frames during one or more sweeps of a blind sweep protocol on a patient with a pregnancy; dividing the first plurality of ultrasound image frames into a plurality of sub-sweeps; determining, based on the first plurality of ultrasound image frames, a plurality of initial values of a fetal parameter and a plurality of initial values of a confidence for the plurality of sub-sweeps, respectively; determining an initial value of the confidence that is relatively larger than other initial values of the confidence, wherein the initial value of the confidence that is identified is specific to a sub-sweep of the plurality of sub-sweeps; and outputting, to a display, a first screen display comprising an instruction to a user to perform a follow-up scan with the ultrasound probe on the sub-sweep associated to the identified initial value of confidence. The method enables structured guidance for users to perform follow-up scans based on confidence values derived from initial scans, optimizing the overall workflow and ensuring high-quality data acquisition regardless of the user's expertise level.

Another general aspect includes a non-transitory computer-readable medium storing instructions that, when executed by a processor, cause the processor to perform the above method. Storing and executing the method instructions from a non-transitory computer-readable medium may advantageously ensure that the system is scalable, portable, and easily deployable across various devices, enhancing accessibility to advanced ultrasound diagnostics in diverse and resource-constrained settings.

Implementations may include one or more of the following features. In some aspects, the processor circuit is configured to generate a heatmap based on the plurality of initial values of the confidence, wherein the first screen display may include the heatmap. Generating a heatmap from the initial confidence values advantageously allows for intuitive visual guidance, helping users quickly identify high-confidence areas, thereby making the follow-up scanning process more targeted and efficient. In some aspects, the heatmap identifies a location of the sub-sweep. Identifying specific sub-sweep locations on the heatmap may ensure precise targeting for follow-up scans, improving the accuracy and reliability of fetal parameter measurements by focusing on areas where the initial data is most promising. In some aspects, the plurality of sub-sweeps is respectively color-coded in the heatmap based on the plurality of initial values of the confidence. Color-coding sub-sweeps based on confidence values may provide a clear and easily interpretable visual map, which helps users differentiate regions of varying data quality, thus simplifying the decision-making process for follow-up scans. In some aspects, the heatmap may include a graphical representation of the one or more sweeps of the blind sweep protocol. In some aspects, the heatmap may include a graphical presentation of an abdomen of the patient. Displaying the heatmap over a graphical representation of the sweeps or the patient's abdomen may enhance user orientation and ensures that follow-up scans are performed accurately, even by minimally trained users, leading to better data capture and more reliable diagnoses.

In some aspects, the processor circuit is configured to generate one or more statistics based on at least one of: the plurality of initial values of the fetal parameter; or the plurality of initial values of the confidence, wherein the first screen display may include the one or more statistics. Generating and displaying statistical summaries of fetal parameters and/or confidence values may advantageously give users critical insights into the quality and reliability of the scan data, helping them make more informed clinical decisions. In some aspects, a quantity of the first plurality of ultrasound image frames in each sub-sweep of the plurality of sub-sweeps is less than a quantity of the first plurality of ultrasound image frames in a single sweep of the plurality of sweeps. By ensuring that sub-sweeps contain fewer image frames than a full sweep, the apparatus may effectively balance data processing load and memory usage, making the system practical for use on devices with limited computational resources like smartphones and tablets. In some aspects, a quantity of the first plurality of ultrasound image frames in each of the plurality of sub-sweeps is equal. In some aspects, a quantity of the first plurality of ultrasound image frames in a first sub-sweep and in a second sub-sweep of the plurality of sub-sweeps is different from one another. In some aspects, the processor circuit is further configured to detect one or more fetal anatomies in the first plurality of ultrasound images frames, wherein the first plurality of ultrasound image frames are divided into the plurality of sub-sweeps based on the one or more fetal anatomies. Detecting fetal anatomies within the ultrasound frames and using this information to define sub-sweeps may ensure that each segment of data is relevant and focused on critical areas, enhancing the precision and accuracy of fetal parameter determinations. In some aspects, the processor circuit is configured to: provide the first plurality of ultrasound image frames as an input to an object detection neural network trained to detect the one or more fetal anatomies; and detect the one or more fetal anatomies as an output of the object detection neural network.

In some aspects, the processor circuit is configured to control the ultrasound probe to obtain a second portion of the first plurality of ultrasound image frames simultaneously as at least one: the processor circuit assigning a first portion of the first plurality of ultrasound image frames to a given sub-sweep; or the processor circuit determining, using the first portion of the first plurality of ultrasound image frames, a given initial value of the fetal parameter and a given initial value of the confidence for the given sub-sweep. Simultaneously processing ultrasound image frames while acquiring new data may advantageously allow for real-time or near-real-time refinement of sub-sweeps and the related fetal parameter predictions, thereby optimizing the efficiency and effectiveness of the scanning protocol. In some aspects, the processor circuit is configured to: control the ultrasound probe to obtain a second plurality of ultrasound image frames during the follow-up scan on the sub-sweep; determine, based on the second plurality of ultrasound image frames, a follow-up value of the fetal parameter and a follow-up value of the confidence for the follow-up scan; and output, to the display, a second screen display may include at least one of: the follow-up value of the fetal parameter; or the follow-up value of the confidence. In some aspects, a quantity of the second plurality of ultrasound image frames in the follow-up scan is greater than the quantity of the first plurality of ultrasound image frames in the sub-sweep. Conducting follow-up scans with increased frame density in high-confidence sub-sweep areas may facilitate higher resolution and more detailed data capture, resulting in significantly more accurate fetal parameter and confidence values.

In some aspects, the processor circuit is configured to determine a further initial value of the confidence that is relatively larger than the other initial values of the confidence, wherein the further initial value of the confidence that is identified is specific to a further sub-sweep of the plurality of sub-sweeps; wherein the instruction to the user is to perform a further follow-up scan with the ultrasound probe on the further sub-sweep. By identifying and re-scanning further high-confidence areas, the apparatus may continuously improve the accuracy and robustness of fetal parameter measurements, ensuring that critical diagnostic information is as reliable as possible. In some aspects, the second plurality of ultrasound image frames may include the further follow-up scan on the further sub-sweep, wherein the processor circuit is configured to: determine, based on the second plurality of ultrasound image frames, a further follow-up value of the fetal parameter and a further follow-up value of the confidence for the further follow-up scan; and generate one or more statistics based on at least one of: the follow-up value of the fetal parameter for the follow-up scan and the further follow-up value of the fetal parameter for the further follow-up scan; or the follow-up value of the confidence for the follow-up scan and the further follow-up value of the confidence for the further follow-up scan, and wherein the second screen display may include the one or more statistics. Aggregating follow-up values for both fetal parameters and their confidences across multiple scans may beneficially provide a comprehensive and statistically robust set of data, enhancing the reliability of diagnostic results and supporting better clinical decision-making.

In some aspects, the one or more sweeps may include one or more longitudinal movements with the ultrasound probe on the patient body, and wherein the follow-up scan may include an additional longitudinal movement or a non-longitudinal movement with the ultrasound probe on the patient body. In some aspects, the processor circuit is configured to: provide the first plurality of ultrasound image frames as an input to a regression neural network trained to determine the plurality of initial values of the fetal parameter and the plurality of initial values of the confidence; and determine the plurality of initial values of the fetal parameter and the plurality of initial values of the confidence as an output of the regression neural network. Utilizing a regression neural network for predicting initial values of fetal parameters and confidences may advantageously leverage advanced machine learning techniques to provide high accuracy and reliability in fetal parameter estimations from blind sweep data. In some aspects, the apparatus may include a tablet or a smartphone, which may include the processor circuit such that the processor circuit is not part of a dedicated ultrasound console. Implementations of the described techniques may include hardware, a method or process, or computer software on a computer-accessible medium.

This Summary is provided to introduce a selection of concepts in a simplified form that are further described below in the Detailed Description. This Summary is not intended to identify key features or essential features of the claimed subject matter, nor is it intended to limit the scope of the claimed subject matter. A more extensive presentation of features, details, utilities, and advantages of the ultrasound sweep fetal parameter detection system, as defined in the claims, is provided in the following written description of various aspects of the disclosure and illustrated in the accompanying drawings.

### BRIEF DESCRIPTION OF THE DRAWINGS

Illustrative aspects of the present disclosure will be described with reference to the accompanying drawings, of which:
Fig. 1 is a schematic, diagrammatic representation of an ultrasound imaging system, according to aspects of the present disclosure.
Fig. 2 is a schematic diagram of a processor circuit, according to aspects of the present disclosure.
Fig. 3 is a schematic, diagrammatic representation of a patient, according to aspects of the present disclosure.
Fig. 4A is an exemplary workflow for a blind sweep protocol, according to aspects of the present disclosure.
Fig. 4B is an exemplary workflow for a blind sweep protocol, according to aspects of the present disclosure.
Fig. 4C is an exemplary workflow for a blind sweep protocol, according to aspects of the present disclosure.
Fig. 4D is an exemplary workflow for a blind sweep protocol, according to aspects of the present disclosure.
Fig. 5 is a high-level block diagram of the blind sweep fetal parameter detection system, according to aspects of the present disclosure.
Fig. 6 is a high-level flow diagram of a blind sweep fetal parameter detection method, according to aspects of the present disclosure.
Fig. 7 is a schematic, diagrammatic representation, in flow diagram form, of an example blind sweep fetal parameter detection method, according to aspects of the present disclosure.
Fig. 8 is a schematic, diagrammatic representation, in block diagram form, of at least a portion of an example ultrasound sweep fetal parameter detection system, according to aspects of the present disclosure.
Fig. 9 is a schematic, diagrammatic representation, in block diagram form, of at least a portion of an example ultrasound sweep fetal parameter detection system, according to aspects of the present disclosure.
Fig. 10 is a screen display of an example ultrasound sweep fetal parameter detection system, according to aspects of the present disclosure.
Fig. 11 is a screen display of an example ultrasound sweep fetal parameter detection system, according to aspects of the present disclosure.
Fig. 12 is a screen display of an example ultrasound sweep fetal parameter detection system, according to aspects of the present disclosure.
Fig. 13 is a schematic, diagrammatic representation, in flow diagram form, of an example ultrasound sweep fetal parameter detection method, according to aspects of the present disclosure.
Fig. 14 is a schematic, diagrammatic representation of a follow-up scanning process 1400, according to aspects of the present disclosure.
Fig. 15 is a schematic, diagrammatic representation, in block diagram form, of at least a portion of an example ultrasound sweep fetal parameter detection system, according to aspects of the present disclosure.
Fig. 16 is screen display for an example ultrasound sweep fetal parameter detection system, according to aspects of the present disclosure.
Fig. 17 is a schematic, diagrammatic representation of the division of sweeps into sub-sweeps of variable size, according to aspects of the present disclosure.
Fig. 18 is a schematic, diagrammatic representation, in flow diagram form, of an example ultrasound sweep fetal parameter detection method, according to aspects of the present disclosure.
Fig. 19 is a schematic, diagrammatic representation of the division of sweeps into sub-sweeps of variable size, according to aspects of the present disclosure.
Fig. 20 is a schematic, diagrammatic representation of the division of sweeps into sub-sweeps of variable size, according to aspects of the present disclosure.
Fig. 21 is a schematic, diagrammatic representation, in flow diagram form, of an example ultrasound sweep fetal parameter detection method, according to aspects of the present disclosure.
Fig. 22 is a schematic, diagrammatic representation, in flow diagram form, of an example real-time or near-real time ultrasound sweep fetal parameter detection method, according to aspects of the present disclosure.

### DETAILED DESCRIPTION OF EMBODIMENTS

In accordance with at least one aspect of the present disclosure, an ultrasound sweep fetal parameter detection system is provided which can make and refine fetal parameter predictions based on a blind sweep protocol with an ultrasound imaging probe. The ultrasound sweep fetal parameter detection system presents a novel approach to imaging quality control by ensuring that high-confidence regions are re-scanned at higher resolution, thus leading to follow-up fetal parameter predictions that have higher confidence and accuracy. Fetal parameters may for example include gestational age, fetal presentation or lie, detecting multiple pregnancies, measuring the deepest vertical pocket of amniotic fluid, identifying a low-lying placenta and placenta previa, cardiac activity, fetal heart rate, confirming intrauterine pregnancy, measuring placental volume, detecting fetal anomalies, etc.

Ultrasound imaging is a vital component of high-quality obstetric care. In rural and under-resourced communities, the scarcity of ultrasound imaging results in a considerable gap in healthcare for pregnant mothers. Improved detection of fetal parameters through ultrasound can allow for appropriate and timely referral for delivery care in more-resourced centers with more highly trained providers. The present disclosure seeks to overcome this barrier to ultrasound access in a locally sustainable and resource-conscious way, using standardized blind-sweep scanning protocols combined with artificial intelligence, obviating the need for an interpreting provider (e.g., a radiologist or obstetrician) and an experienced sonographer in the remote location.

In low resource ultrasound settings (e.g., low- and middle-income countries), it is often not possible to run the standard clinical workflow for fetal biometry workup, primarily due to lack of adequate clinical expertise in acquiring and interpreting the ultrasound scans, and secondly also due to lack of access to ultrasound equipment. Blind sweep protocols enable a novice or minimally trained ultrasound user to conduct an elementary screening for automated estimation of basic but important parameters like gestational age, fetal presentation, cardiac activity, multiple gestation, placenta location and deepest vertical pocket of amniotic fluid. Efforts are underway to integrate these capabilities into a handheld ultrasound platform (e.g., ultrasound probe connected to a tablet or phone), to enable easier uptake of the solution in low resource settings.

Aspects of the present application (e.g., a deep learning model trained to estimate a clinical feature) can include or be similar to those described in U.S. Provisional Application No. 63/611,810, filed December 19, 2023, titled "Low-Lying Placenta and/or Placenta Location in Ultrasound Imaging With Blind Sweep Protocol", U.S. Provisional Application No. 63/540,755, filed September 27, 2023, titled "Ultrasound Imaging With Follow Up Sweep Guidance After Blind Sweep Protocol", U.S. Provisional Application No. 63/655,754, filed June 4, 2024, titled "Multiple Pregnancy/Gestation Detection Based On Graphing And Skeletonization Of Fetal Anatomy Detections From Ultrasound Imaging Blind Sweep Protocol", U.S. Provisional Application No. 63/620,385, filed January 12, 2024, titled "Multiple Pregnancy/Gestation Detection Using Ultrasound Imaging With Blind Sweep Protocol", and U.S. Provisional Application No. 63/540,740, filed September 27, 2023, titled "Ultrasound Imaging With Ultrasound Probe Guidance In Blind Sweep Protocol", and U.S. Provisional Application No. 63/690,889, filed September 5, titled "Ultrasound-Based Verification Of Uterine Imaging Extent Using Blind Sweep Protocol", each of which is incorporated by reference herein. Another example of determine a fetal parameter, such as gestational age (GA), is described in U.S. Provisional Application No. 63/751,925, filed January 31, 2025, titled "Standard Imaging Plane Detection In Ultrasound Imaging With Blind Sweep Protocol", which is incorporated by reference herein.

Other examples of measuring fetal parameters, such as gestational age (GA), using deep learning include "AI Estimation of Gestational Age from Blind Ultrasound Sweeps in Low-Resource Settings", NEJM Evidence, and "A mobile-optimized artificial intelligence system for gestational age and fetal malpresentation assessment", Communications Medicine, each of which are incorporated by reference herein.

However, while the full dataset of a blind sweep protocol can be used to generate fetal parameters with high confidence and accuracy, such a comprehensive analysis may rely on fast processors and/or processors that include a large amount of memory. Because the blind sweep protocol is intended for low-resource environments and equipment, blind sweep analysis methods can produce outlier measurements, compromising accuracy. For example, due to resource constraints (e.g., memory-related performance constraints on mobile devices such as tablets or phones), blind sweep analysis methods are designed for use with such low-resource equipment, and thus process only a small subset of the total collected imaging data, which often can result in outlier measurements which are not correct. For those situations, currently there is no mitigation.

To address this limitation, the present disclosure introduces a novel confidence refinement sweep workflow that enhances the accuracy of output fetal parameters, also known as fetal indicators. This workflow involves dividing blind sweep data into sub-sweeps, estimating confidence and gestational age or other fetal parameters using neural networks, and highlighting high-confidence sub-sweeps on an anatomy map. Users are then prompted to conduct follow-up targeted sweeps around regions of interest (e.g., the high-confidence regions), acquiring additional data that is fed into the same or another neural network to re-estimate the fetal parameters. This innovative approach improves the accuracy of fetal parameters, mitigating the limitations of blind sweep algorithms in low-resource settings. The workflow is supported by a unique user interface and audio-visual prompt system, enabling users to efficiently refine their measurements and improve patient outcomes.

In the context of the present disclosure, the confidence of a fetal parameter prediction (i.e., the confidence of a value of the fetal parameter) in a given sub-sweep preferably refers to a score or a probability describing the likelihood of determining, based on the ultrasound image frames comprised in said sub-sweep, the value of the fetal parameter with a level of accuracy equal to or larger than a predetermined accuracy threshold. Also in the context of the present disclosure, the accuracy of a fetal parameter prediction preferably refers to a score or a probability describing the likelihood that the determined value of the fetal parameter is correct.

Problems or disadvantages overcome by the invention include overcoming outlier measurements. Current methods can produce outlier measurements due to resource constraints (e.g., smaller chunks of data being fed to the models, to ensure manageable run-times), leading to incorrect results. There is currently no effective way to mitigate these outlier measurements, resulting in inaccurate fetal parameters. For example, blind sweeps may not provide accurate gestational age estimation, which is crucial for fetal development monitoring. Current workflows also do not provide a mechanism for targeted follow-up scans, which can lead to missed opportunities for detecting standard planes for gestational age (GA) measurement, or detection of fetal abnormalities. Current workflows also do not provide a mechanism for refining estimates based on user input, leading to potential errors in fetal parameter estimation.

Element(s) of the invention include dividing the blind sweep data into sub-sweeps of e.g., 40 frames each, to avoid overstressing detection tools. Each of the sub-sweeps are then passed to the downstream tools like gestational age or fetal presentation detectors, and the neural network regressor model outputs the estimated confidence and the estimated gestational age (or other fetal parameter) value for each sub-sweep. The sub-sweeps having the highest confidence are highlighted on an anatomy map. The user is then prompted by audio/visual prompts to do a follow up scan around the highlighted region of interest for a fixed duration. The data acquired from the follow up scan is then passed through a the same or another neural network (or multiple neural networks) to estimate the appropriate fetal parameters. The novel workflow thus includes a pre-screening scan which could be blind sweeps, then running models on the pre-screening scan, followed by a follow-up scan for higher resolution which has more frames in the target area where the confidence of the model was the highest.

In one exemplary aspect, the follow-up scan is based on a confidence heatmap of the initial model outputs. In this example, the user collects the blind sweep data, and then the blind sweep data is divided into sub-sweeps and passed through neural network regressors or classifiers, which output the fetal parameter and confidence. The confidence and fetal parameters are then displayed onto a heatmap which shows the areas with the highest confidence. The user interface prompts the user via audiovisual prompts to perform a follow-up scan on the area of interest.

The user performs the follow-up scan, and the neural network model again runs on the collected data to estimate the fetal parameters with a higher confidence. Alternately, the new (richer) data can be subject to other types of estimation methods. In some aspects, the higher confidence region that is identified can be one that includes a standard plane. The Performing the follow-up scan in that region allows for collections of ultrasound imaging planes close to the standard plane (e.g., +/-5° or +/- 5 mm) or maybe even the standard plane itself. Typically, in a blind sweep protocol, a standard plane is not intentionally searched for. However, it is still possible (either in the blind sweep protocol or the follow-up scan) to obtain a standard plane or imaging plane close to the standard plane without the processor actually identifying the obtained plane as the standard plane. Determining the fetal parameter using ultrasound imaging planes close to the standard plane or including the standard plane can increase the accuracy of the determined fetal parameter. In other aspects, an intentional search for the standard plane can be conducted in the context of a blind sweep protocol. One example is to look for the presence of fetal standard planes, and if present, trigger a standard plane-based GA calculation approach or similar methods for other fetal parameters. Identifying a standard plane in the context of a blind sweep protocol and determining a gestational age (GA) is described in U.S. Provisional Application No. 63/751,925, filed January 31, 2025, titled "Standard Imaging Plane Detection In Ultrasound Imaging With Blind Sweep Protocol", which is incorporated by reference herein.

In another exemplary aspect, the user interface (UI) may show the confidence heatmap with a varying color scheme for different sub-sweeps with differing confidences (e.g., one color or brightness for high confidence, and another color or brightness for low confidence, with a spectrum of values in between). The UI then gives audiovisual (A/V) prompts to the user to trigger a follow up scan on areas of interest, using the sub-sweeps themselves as a reference, or using a representation of the patient's anatomy. The UI can also output the fetal parameters and the confidence of the fetal parameters, possibly along with statistics such as the average confidence across sub-sweeps, or the highest confidence of a given sub-sweep.

In still another exemplary aspect, the system may perform sub-sweep division and real-time inference in real time or near-real time, while acquiring the ultrasound images. In this aspect, the sweeps can be divided into any arbitrary number of sub-sweeps, with fixed or variable sub-sweep lengths. The sub-sweep division can be done after all the sweeps are acquired and then passed to the neural networks, or the sub-sweeps may be passed as they are being acquired live to the neural networks. The sub-sweep division can also be on the basis of anatomy detections by a separate model, and the sub-sweeps could be divided in such a way that they contain full anatomies of the fetus, with one sub-sweep having only one anatomy of interest. Another way of dividing is to ensure that each sub-sweep has multiple anatomies in it, such that in each run, the model will see a combination of different anatomies.

In still another exemplary aspect, aggregation of outputs is performed on the basis of sub-sweep confidences. In this example, the neural network model outputs on sub-sweeps could be weighted by their confidences and shown on screen as the weighted-average fetal parameter. High confidence regions could be used to trigger multiple follow-up scans to get a more accurate output.

In still another exemplary aspect, the confidence value of any fetal parameter could be a softmax output of a neural network. The confidence could, alternatively or in addition, be the inverse of the variance prediction of the neural network where the variance is calculated through softplus functions, or other functions known in the art. The confidence could also be the number of fetal anatomies detected in a particular region.

The present disclosure aids substantially in the capture of high-quality ultrasound images by minimally trained users, by detecting fetal parameters and confidence values, and requiring that the user re-scan areas where the estimated confidence is highest (e.g., regions where the fetal anatomy most lends itself to accurate prediction of the fetal parameters). Implemented on a processor in communication with an ultrasound probe, the ultrasound sweep fetal parameter detection system disclosed herein provides practical improvements in the diagnosis, treatment, and prophylaxis of pregnancy-related conditions, and of the quality of care available to patients in underserved areas. This improved imaging methodology transforms a process that is heavily reliant on professional experience into one that is accurate and repeatable even for minimally trained personnel, without the normally routine need to train clinicians such as emergency department personnel to estimate fetal parameters. This unconventional approach improves the functioning of the ultrasound imaging system, by providing reliable, repeatable fetal parameter estimation in hospital, office, vehicle, field, and home settings, as well as referral recommendations for patients for whom the fetal parameters indicate a pregnancy complication.

The ultrasound sweep fetal parameter detection system may be implemented as a process at least partially viewable on a display, and operated by a control process executing on a processor that accepts user inputs from a keyboard, mouse, or touchscreen interface, and that is in communication with one or more sensor probes. In that regard, the control process performs certain specific operations in response to different inputs or selections made at different times. Certain structures, functions, and operations of the processor, display, sensors, and user input systems are known in the art, while others are recited herein to enable novel features or aspects of the present disclosure with particularity.

These descriptions are provided for exemplary purposes only, and should not be considered to limit the scope of the ultrasound sweep fetal parameter detection system. Certain features may be added, removed, or modified without departing from the spirit of the claimed subject matter.

For the purposes of promoting an understanding of the principles of the present disclosure, reference will now be made to the aspects illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one aspect may be combined with the features, components, and/or steps described with respect to other aspects of the present disclosure. For the sake of brevity, however, the numerous iterations of these combinations will not be described separately.

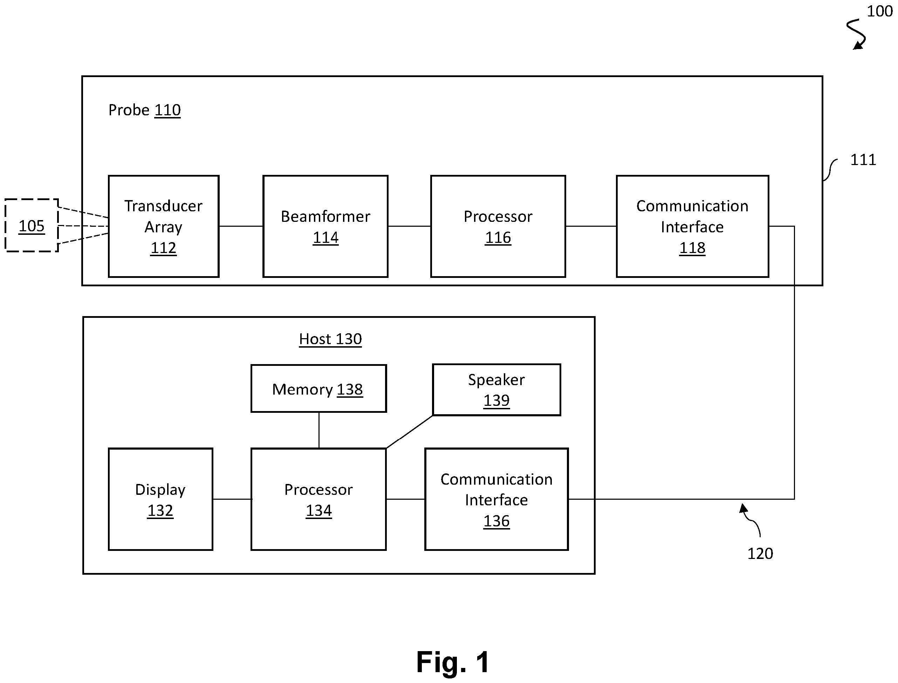

Fig. 1 is a schematic, diagrammatic representation of an ultrasound imaging system 100, according to aspects of the present disclosure. The ultrasound imaging system 100 may for example be used to acquire ultrasound video sweeps, which can then be analyzed by a human clinician or an artificial intelligence to diagnose medical conditions.

The ultrasound imaging system 100 is used for scanning an area or volume of a subject's body. A subject may include a patient of an ultrasound imaging procedure, or any other person, or any suitable living or non-living organism or structure. The ultrasound imaging system 100 includes an ultrasound imaging probe 110 in communication with a host 130 over a communication interface or link 120. The probe 110 may include a transducer array 112, a beamformer 114, a processor circuit 116, and a communication interface 118. The host 130 may include a display 132, a processor circuit 134, a communication interface 136, and a memory 138 storing subject information.

In some aspects, the probe 110 is an external ultrasound imaging device including a housing 111 configured for handheld operation by a user. The transducer array 112 can be configured to obtain ultrasound data while the user grasps the housing 111 of the probe 110 such that the transducer array 112 is positioned adjacent to or in contact with a subject's skin. The probe 110 is configured to obtain ultrasound data of anatomy within the subject's body while the probe 110 is positioned outside of the subject's body for general imaging, such as for abdomen imaging, liver imaging, etc. In some aspects, the probe 110 can be an external ultrasound probe, a transthoracic probe, and/or a curved array probe.

In other aspects, the probe 110 can be an internal ultrasound imaging device and may comprise a housing 111 configured to be positioned within a lumen of a subject's body for general imaging, such as for abdomen imaging, liver imaging, etc.. In some aspects, the probe 110 may be a curved array probe. Probe 110 may be of any suitable form for any suitable ultrasound imaging application including both external and internal ultrasound imaging.

In some aspects, aspects of the present disclosure can be implemented with medical images of subjects obtained using any suitable medical imaging device and/or modality. Examples of medical images and medical imaging devices include x-ray images (angiographic images, fluoroscopic images, images with or without contrast) obtained by an x-ray imaging device, computed tomography (CT) images obtained by a CT imaging device, positron emission tomography-computed tomography (PET-CT) images obtained by a PET-CT imaging device, magnetic resonance images (MRI) obtained by an MRI device, single-photon emission computed tomography (SPECT) images obtained by a SPECT imaging device, optical coherence tomography (OCT) images obtained by an OCT imaging device, and intravascular photoacoustic (IVPA) images obtained by an IVPA imaging device. The medical imaging device can obtain the medical images while positioned outside the subject body, spaced from the subject body, adjacent to the subject body, in contact with the subject body, and/or inside the subject body.

For an ultrasound imaging device, the transducer array 112 emits ultrasound signals towards an anatomical object 105 of a subject and receives echo signals reflected from the object 105 back to the transducer array 112. The ultrasound transducer array 112 can include any suitable number of acoustic elements, including one or more acoustic elements and/or a plurality of acoustic elements. In some instances, the transducer array 112 includes a single acoustic element. In some instances, the transducer array 112 may include an array of acoustic elements with any number of acoustic elements in any suitable configuration. For example, the transducer array 112 can include between 1 acoustic element and 10000 acoustic elements, including values such as 2 acoustic elements, 4 acoustic elements, 36 acoustic elements, 64 acoustic elements, 128 acoustic elements, 500 acoustic elements, 812 acoustic elements, 1000 acoustic elements, 3000 acoustic elements, 8000 acoustic elements, and/or other values both larger and smaller. In some instances, the transducer array 112 may include an array of acoustic elements with any number of acoustic elements in any suitable configuration, such as a linear array, a planar array, a curved array, a curvilinear array, a circumferential array, an annular array, a phased array, a matrix array, a one-dimensional (1D) array, a 1.x dimensional array (e.g., a 1.5D array), or a two-dimensional (2D) array. The array of acoustic elements (e.g., one or more rows, one or more columns, and/or one or more orientations) can be uniformly or independently controlled and activated. The transducer array 112 can be configured to obtain one-dimensional, two-dimensional, and/or three-dimensional images of a subject's anatomy. In some aspects, the transducer array 112 may include a piezoelectric micromachined ultrasound transducer (PMUT), capacitive micromachined ultrasonic transducer (CMUT), single crystal, lead zirconate titanate (PZT), PZT composite, other suitable transducer types, and/or combinations thereof.

The object 105 may include any anatomy or anatomical feature, such as a kidney, liver, and/or any other anatomy of a subject. The present disclosure can be implemented in the context of any number of anatomical locations and tissue types, including without limitation, organs including the liver, kidneys, gall bladder, pancreas, lungs; ducts; intestines; nervous system structures including the brain, dural sac, spinal cord and peripheral nerves; the urinary tract; as well as valves within the blood vessels, blood, abdominal organs, and/or other systems of the body. In some aspects, the object 105 may include malignancies such as tumors, cysts, lesions, hemorrhages, or blood pools within any part of human anatomy. The anatomy may be a blood vessel, as an artery or a vein of a subject's vascular system, including cardiac vasculature, peripheral vasculature, neural vasculature, renal vasculature, and/or any other suitable lumen inside the body. In addition to natural structures, the present disclosure can be implemented in the context of man-made structures such as, but without limitation, heart valves, stents, shunts, filters, implants and other devices.

The beamformer 114 is coupled to the transducer array 112. The beamformer 114 controls the transducer array 112, for example, for transmission of the ultrasound signals and reception of the ultrasound echo signals. In some aspects, the beamformer 114 may apply a time-delay to signals sent to individual acoustic transducers within an array in the transducer 112 such that an acoustic signal is steered in any suitable direction propagating away from the probe 110. The beamformer 114 may further provide image signals to the processor circuit 116 based on the response of the received ultrasound echo signals. The beamformer 114 may include multiple stages of beamforming. The beamforming can reduce the number of signal lines for coupling to the processor circuit 116. In some aspects, the transducer array 112 in combination with the beamformer 114 may be referred to as an ultrasound imaging component.

The processor 116 is coupled to the beamformer 114. The processor 116 may also be described as a processor circuit, which can include other components in communication with the processor 116, such as a memory, beamformer 114, communication interface 118, and/or other suitable components. The processor 116 may include a central processing unit (CPU), a graphical processing unit (GPU), a digital signal processor (DSP), an application specific integrated circuit (ASIC), a controller, a field programmable gate array (FPGA) device, another hardware device, a firmware device, or any combination thereof configured to perform the operations described herein. The processor 116 may also be implemented as a combination of computing devices, e.g., a combination of a DSP and a microprocessor, a plurality of microprocessors, one or more microprocessors in conjunction with a DSP core, or any other such configuration. The processor 116 is configured to process the beamformed image signals. For example, the processor 116 may perform filtering and/or quadrature demodulation to condition the image signals. The processor 116 and/or 134 can be configured to control the array 112 to obtain ultrasound data associated with the object 105.

The communication interface 118 is coupled to the processor 116. The communication interface 118 may include one or more transmitters, one or more receivers, one or more transceivers, and/or circuitry for transmitting and/or receiving communication signals. The communication interface 118 can include hardware components and/or software components implementing a particular communication protocol suitable for transporting signals over the communication link 120 to the host 130. The communication interface 118 can be referred to as a communication device or a communication interface module.
The communication link 120 may be any suitable communication link. For example, the communication link 120 may be a wired link, such as a universal serial bus (USB) link or an Ethernet link. Alternatively, the communication link 120 may be a wireless link, such as an ultra-wideband (UWB) link, an Institute of Electrical and Electronics Engineers (IEEE) 802.11 WiFi link, or a Bluetooth link.

At the host 130, the communication interface 136 may receive the image signals. The communication interface 136 may be substantially similar to the communication interface 118. The host 130 may be any suitable computing and display device, such as a workstation, a personal computer (PC), a laptop, a tablet, or a mobile phone.

The processor 134 is coupled to the communication interface 136. The processor 134 may also be described as a processor circuit, which can include other components in communication with the processor 134, such as the memory 138, the communication interface 136, an optional speaker 139, and/or other suitable components. The processor 134 may be implemented as a combination of software components and hardware components. The processor 134 may include a central processing unit (CPU), a graphics processing unit (GPU), a digital signal processor (DSP), an application-specific integrated circuit (ASIC), a controller, an FPGA device, another hardware device, a firmware device, or any combination thereof configured to perform the operations described herein. The processor 134 may also be implemented as a combination of computing devices, e.g., a combination of a DSP and a microprocessor, a plurality of microprocessors, one or more microprocessors in conjunction with a DSP core, or any other such configuration. The processor 134 can be configured to generate image data from the image signals received from the probe 110. The processor 134 can apply advanced signal processing and/or image processing techniques to the image signals. In some aspects, the processor 134 can form a three-dimensional (3D) volume image from the image data. In some aspects, the processor 134 can perform real-time processing on the image data to provide a streaming video of ultrasound images of the object 105. In some aspects, the host 130 includes a beamformer. For example, the processor 134 can be part of and/or otherwise in communication with such a beamformer. The beamformer in the in the host 130 can be a system beamformer or a main beamformer (providing one or more subsequent stages of beamforming), while the beamformer 114 is a probe beamformer or micro-beamformer (providing one or more initial stages of beamforming).

The memory 138 is coupled to the processor 134. The memory 138 may be any suitable storage device, such as a cache memory (e.g., a cache memory of the processor 134), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, solid state drives, other forms of volatile and non-volatile memory, or a combination of different types of memory.

The memory 138 can be configured to store subject information, measurements, data, or files relating to a subject's medical history, history of procedures performed, anatomical or biological features, characteristics, or medical conditions associated with a subject, computer readable instructions, such as code, software, or other application, as well as any other suitable information or data. The memory 138 may be located within the host 130. Subject information may include measurements, data, files, other forms of medical history, such as but not limited to ultrasound images, ultrasound videos, and/or any imaging information relating to the subject's anatomy. The subject information may include parameters related to an imaging procedure such as an anatomical scan window, a probe orientation, and/or the subject position during an imaging procedure. The memory 138 can also be configured to store information related to the training and implementation of machine learning algorithms (e.g., neural networks) and/or information related to implementing image recognition algorithms for detecting/segmenting anatomy, image quantification algorithms, and/or image acquisition guidance algorithms, including those described herein.

The display 132 is coupled to the processor circuit 134. The display 132 may be a monitor or any suitable display. The display 132 is configured to display the ultrasound images, image videos, and/or any imaging information of the object 105.

The ultrasound imaging system 100 may be used to assist a sonographer in performing an ultrasound scan. The scan may be performed in a point-of-care setting. In some instances, the host 130 is a console or movable cart. In some instances, the host 130 may be a mobile device, such as a tablet, a mobile phone, or portable computer. During an imaging procedure, the ultrasound system can acquire an ultrasound image of a particular region of interest within a subject's anatomy. The ultrasound imaging system 100 may then analyze the ultrasound image to identify various parameters associated with the acquisition of the image such as the scan window, the probe orientation, the subject position, and/or other parameters. The ultrasound imaging system 100 may then store the image and these associated parameters in the memory 138. At a subsequent imaging procedure, the ultrasound imaging system 100 may retrieve the previously acquired ultrasound image and associated parameters for display to a user which may be used to guide the user of the ultrasound imaging system 100 to use the same or similar parameters in the subsequent imaging procedure, as will be described in more detail hereafter.

In some aspects, the processor 134 may utilize deep learning-based prediction networks to identify parameters of an ultrasound image, including an anatomical scan window, probe orientation, subject position, identify and location of anatomical features, and/or other parameters. In some aspects, the processor 134 may receive metrics or perform various calculations relating to the region of interest imaged or the subject's physiological state during an imaging procedure. These metrics and/or calculations may also be displayed to the sonographer or other user via the display 132.

In some aspects, the host 130 may also include a speaker 180. The speaker 180 may for example be used to provide advisory tones, beeps, or other auditory feedback to the user.

Before continuing, it should be noted that the examples described above are provided for purposes of illustration, and are not intended to be limiting. Other devices and/or device configurations may be utilized to carry out the operations described herein.

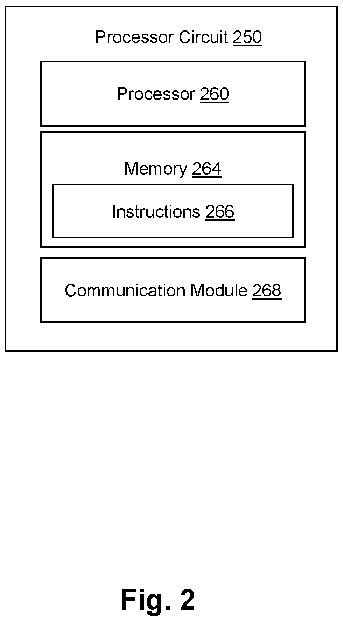

Fig. 2 is a schematic diagram of a processor circuit 250, according to aspects of the present disclosure. The processor circuit 250 may be implemented in the ultrasound imaging system 100, or other devices or workstations (e.g., third-party workstations, network routers, etc.), or on a cloud processor or other remote processing unit, as necessary to implement the method. As shown, the processor circuit 250 may include a processor 260, a memory 264, and a communication module 268. These elements may be in direct or indirect communication with each other, for example via one or more buses.

The processor 260 may include a central processing unit (CPU), a digital signal processor (DSP), a controller, or any combination of general-purpose computing devices, reduced instruction set computing (RISC) devices, application-specific integrated circuits (ASICs), field programmable gate arrays (FPGAs), or other related logic devices, including mechanical and quantum computers. The processor 260 may also comprise another hardware device, a firmware device, or any combination thereof configured to perform the operations described herein. The processor 260 may also be implemented as a combination of computing devices, e.g., a combination of a DSP and a microprocessor, a plurality of microprocessors, one or more microprocessors in conjunction with a DSP core, or any other such configuration.

The memory 264 may include a cache memory (e.g., a cache memory of the processor 260), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an aspect, the memory 264 includes a non-transitory computer-readable medium. The memory 264 may store instructions 266. The instructions 266 may include instructions that, when executed by the processor 260, cause the processor 260 to perform the operations described herein. Instructions 266 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements.

The communication module 268 can include any electronic circuitry and/or logic circuitry to facilitate direct or indirect communication of data between the processor circuit 250, and other processors or devices. In that regard, the communication module 268 can be an input/output (I/O) device. In some instances, the communication module 268 facilitates direct or indirect communication between various elements of the processor circuit 250 and/or the ultrasound imaging system 100. The communication module 268 may communicate within the processor circuit 250 through numerous methods or protocols. Serial communication protocols may include but are not limited to United States Serial Protocol Interface (US SPI), Inter-Integrated Circuit (I²C), Recommended Standard 232 (RS-232), RS-485, Controller Area Network (CAN), Ethernet, Aeronautical Radio, Incorporated 429 (ARINC 429), MODBUS, Military Standard 1553 (MIL-STD-1553), or any other suitable method or protocol. Parallel protocols include but are not limited to Industry Standard Architecture (ISA), Advanced Technology Attachment (ATA), Small Computer System Interface (SCSI), Peripheral Component Interconnect (PCI), Institute of Electrical and Electronics Engineers 488 (IEEE-488), IEEE-1284, and other suitable protocols. Where appropriate, serial and parallel communications may be bridged by a Universal Asynchronous Receiver Transmitter (UART), Universal Synchronous Receiver Transmitter (USART), or other appropriate subsystem.

External communication (including but not limited to software updates, firmware updates, model sharing between the processor and central server, or readings from the ultrasound imaging system 100) may be accomplished using any suitable wireless or wired communication technology, such as a cable interface such as a universal serial bus (USB), micro USB, Lightning, or FireWire interface, Bluetooth, Wi-Fi, ZigBee, Li-Fi, or cellular data connections such as 2G/GSM (global system for mobiles) , 3G/UMTS (universal mobile telecommunications system), 4G, long term evolution (LTE), WiMax, or 5G. For example, a Bluetooth Low Energy (BLE) radio can be used to establish connectivity with a cloud service, for transmission of data, and for receipt of software patches. The controller may be configured to communicate with a remote server, or a local device such as a laptop, tablet, or handheld device, or may include a display capable of showing status variables and other information. Information may also be transferred on physical media such as a USB flash drive or memory stick.

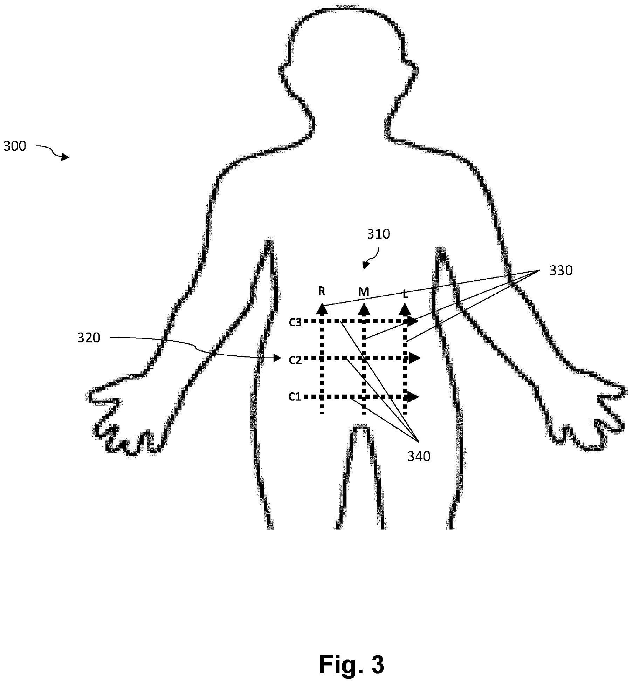

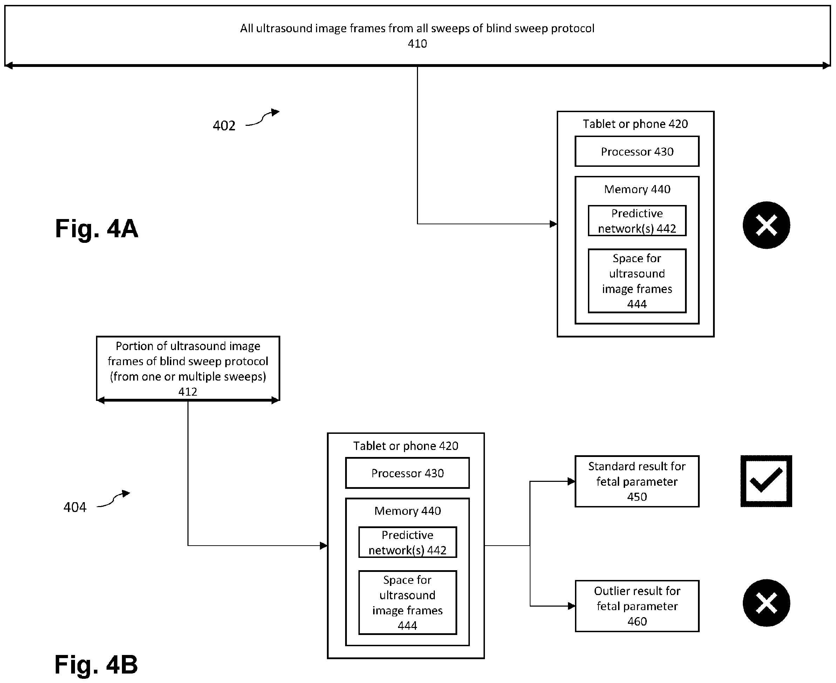

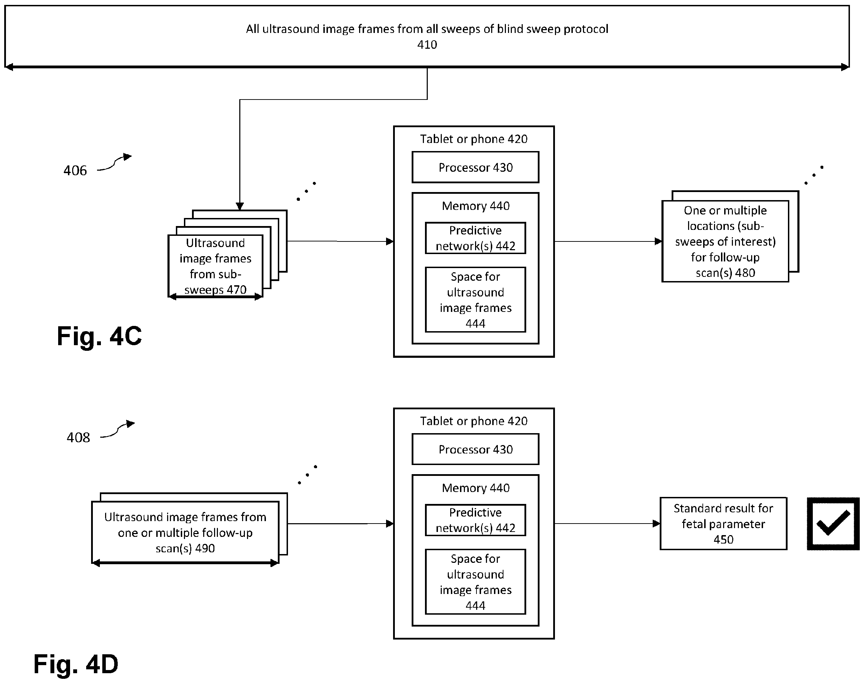

Fig. 3 is a schematic, diagrammatic representation of a patient 300, according to aspects of the present disclosure. Visible on the abdomen 310 of the patient 300 is a desired sweep pattern 320 intended to capture images of desired features of the patient's anatomy. The sweep pattern 320 includes multiple vertical sweep lines 330 and multiple horizontal sweep lines 340. Each sweep line 330, 340 represents a desired path for one imaging sweep of the abdomen 310. In the example shown in Fig. 4, the sweep pattern includes three vertical sweep lines 330 labeled L (patient's left), M (patient's middle), and R (patient's right), all in an upward direction with respect to the patient, and three horizontal sweep lines 340 labeled C1 (bottom), C2 (middle), and C3 (top), all in a right-to-left direction with respect to the patient. However, it is understood that a sweep pattern 320 may include more or fewer sweep lines 330, including vertical sweep lines 330, horizontal sweep lines 330, or combinations thereof, in any combination of upward, downward, left, or right directions, based on the patient's fundal height. For example, if the patient's belly is bigger in size, more sweeps may be needed. Furthermore, a sweep pattern 320 may cover other portions of the patient's body, including but not limited to the head, neck, spine, limbs, etc. Examples of blind sweep protocol include but are not limited to obstetric sweep imaging (OSI), volume sweep imaging (VSI), 6-Stage, Fetal Age Machine Learning Initiative (FAMLI), and Philips.

These sweep patterns represent desired probe motion information, including desired positions, a desired velocity or velocities, and/or a desired orientation of the ultrasound probe while the ultrasound probe is obtaining a plurality of ultrasound image frames during the sweep. It is noted that the desired sweep patterns or blind sweep protocols stored in a memory of the processor may include only vertical sweeps, only horizontal sweeps, may include a grid (e.g., 3x3, 5x5, etc.) of vertical and horizontal sweeps, and may also include associated parameters such as desired probe motion (e.g., positions, velocities, and/or orientations) stored in the memory. Depending on the implementation, sweeps may include curved, diagonal, and other types of sweeps. The protocols and their associated parameters can for example be based on standards established by authorities in the field (physician organizations, sonographer organizations, etc.), published in scholarly journals/textbooks, etc.

Fig. 4A is an exemplary workflow 402 for a blind sweep protocol, according to aspects of the present disclosure. In the example shown in Fig. 4A, all of the ultrasound image data 410, from all of the sweeps of the blind sweep protocol, are passed to a tablet computer or smartphone 420. This dataset 410 could include roughly 200 frames per sweep for up to 10 sweeps, e.g., up to 2000 ultrasound image frames. The tablet or smartphone 420 includes a processor 430 and memory 440. The memory 440 includes the predictive network(s) 442 (and other related instructions such as the user interface), along with space 444 for the ultrasound image frames. However, in many cases, this workflow is non-functional, as the space 444 for the ultrasound image frames is insufficient to store and/or process the entire ultrasound image dataset 410, and/or the processor 430 is too slow to process the entire dataset 410. This can result in unacceptably long processing times and/or outright freezing or crashing of the tablet or phone 420.

Fig. 4B is an exemplary workflow 404 for a blind sweep protocol, according to aspects of the present disclosure. In the example shown in Fig. 4B, only a small portion or subset 412 (e.g., between 100 and 300 frames) of the ultrasound images from the blind sweep protocol (whether from a single sweep or multiple sweeps) is passed to the tablet or smartphone 420, in order to fit within the limited space 444 for the ultrasound image frames in the memory 440 of the tablet or smartphone 420. However, this subset 412 does not necessarily include the image frames most suited for fetal parameter estimation. Thus, based on this portion or subset 412, the predictive network(s) 442 may either produce a standard result 450 for the fetal parameter (indicating a successful completion of the workflow 404, or may produce an outlier result 460 for the fetal parameter, indicating an unsuccessful completion of the workflow 404. Fig. 4B is representative of the way blind sweep protocols currently work for estimation of fetal parameters, and thus shows the vulnerability of current workflows to generating outlier results 460. Fetal parameters may for example include gestational age, fetal presentation or lie, detecting multiple pregnancies, deepest vertical pocket of amniotic fluid, placenta previa, cardiac activity, fetal heart rate, etc.

Fig. 4C is an exemplary workflow 406 for a blind sweep protocol, according to aspects of the present disclosure. In the example shown in Fig. 4C, the entire image dataset 410 from all sweeps of the blind sweep protocol is divided into sub-sweeps 470 of a smaller length (e.g., 40 frames per sub-sweep), that can readily fit into the space 444 for the ultrasound image frames in the memory 440 of the tablet or smartphone 420. The predictive networks 442 then produce estimates of the fetal parameter(s) and associated confidence scores, which are used to identify one or multiple sub-sweeps of interest 480 (e.g., areas of high prediction confidence), whose locations can be re-scanned by the user.

Fig. 4D is an exemplary workflow 408 for a blind sweep protocol, according to aspects of the present disclosure. In the example shown in Fig. 4D, ultrasound image frames from one or multiple follow-up scans 490 of the high-confidence regions are received by the tablet or smartphone 420 and stored in the space 444 for ultrasound image frames. Each of these follow-up scans may cover the same area as the associated high-confidence sub-sweep, but may capture many more image frames (e.g., 200 to 300 image frames, instead of 40 image frames), resulting in higher-resolution coverage of the area of interest. Thus, the predictive network(s) 442 can produce, with high accuracy and confidence, a standard result 450 for the fetal parameter(s). Thus, the improved workflows 406, 408 of Figs. 4C and 4D greatly reduce the chance of an outlier result 460 for the fetal parameter(s), and thus represent a significant improvement in the art of interpreting blind sweep protocols.

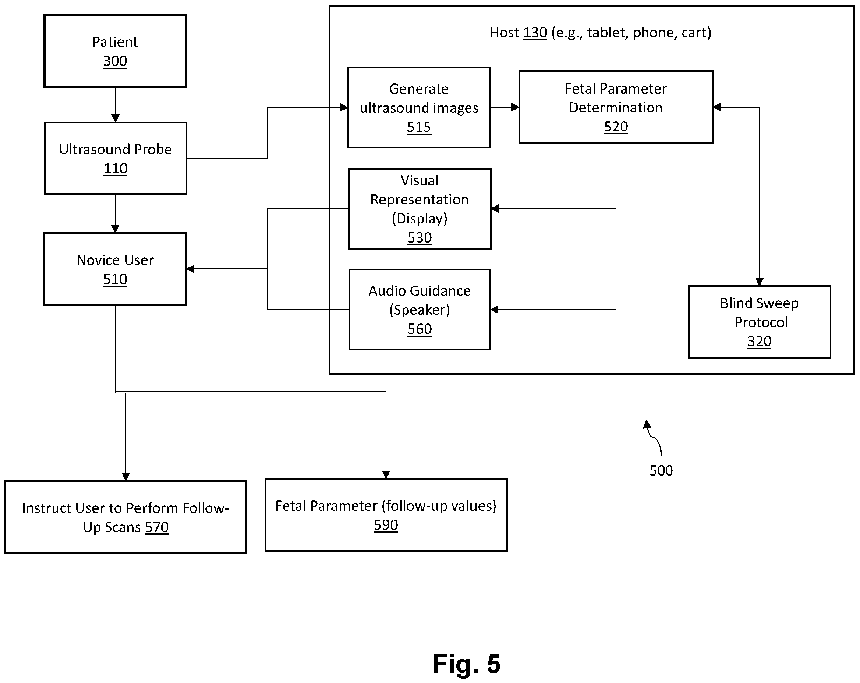

Fig. 5 is a high-level block diagram 500 of the blind sweep fetal parameter detection system, according to aspects of the present disclosure. The patient 300 is scanned with the ultrasound probe 110 by a novice user 510 using a blind sweep protocol 320. The ultrasound probe 110 generates ultrasound images 515, which are used for fetal parameter determination 520. The system then generates a visual representation 530 and/or audio guidance 560, that includes instructing the user to perform follow-up scans 570 on high-confidence areas, resulting in follow-up values 590 for the fetal parameter or parameters.

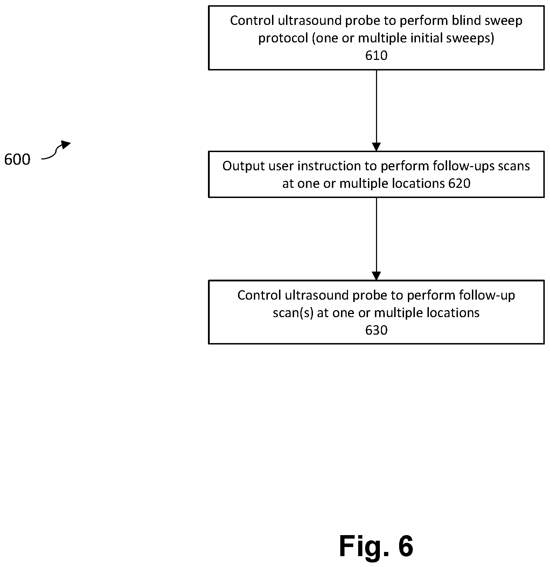

Fig. 6 is a high-level flow diagram of a blind sweep fetal parameter detection method 600, according to aspects of the present disclosure. In step 610, the method 600 includes controlling the ultrasound probe to perform the blind sweep protocol (e.g., one or more initial sweeps). In step 620, the method 600 includes outputting a user instruction to perform follow-up scans at one or multiple locations (e.g., the locations of the highest-confidence sub-scans). In step 630, the method 600 includes controlling the ultrasound probe to perform the follow-up scan(s) at the one or multiple locations.

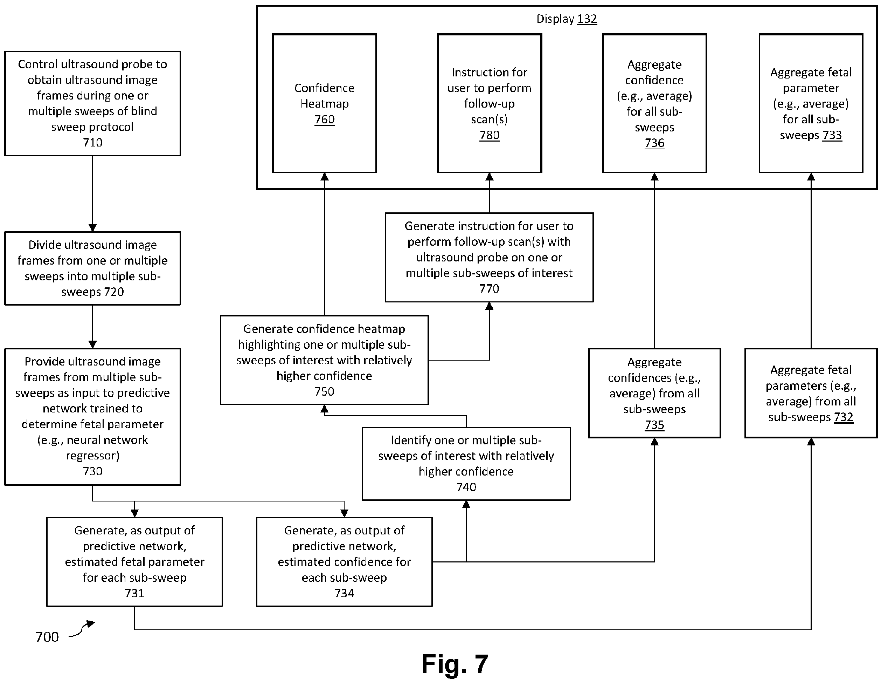

Fig. 7 is a schematic, diagrammatic representation, in flow diagram form, of an example blind sweep fetal parameter detection method 700, according to aspects of the present disclosure. It is understood that the steps of method 700 may be performed in a different order than shown in Fig. 7, additional steps can be provided before, during, and after the steps, and/or some of the steps described can be replaced or eliminated in other embodiments. One or more of steps of the method 700 can be carried by one or more devices and/or systems described herein, such as components of the system 100, tablet or phone 420, and/or processor circuit 250.

In step 710, the method 700 includes controlling the ultrasound probe to obtain ultrasound image frames during one or more sweeps of the blind sweep protocol. Execution then proceeds to step 720.

In step 720, the method 700 includes dividing the ultrasound image frames from the one or multiple sweeps into multiple sub-sweeps. Execution then proceeds to step 730.

In step 730, the method 700 includes providing the ultrasound image frames from the multiple sub-sweeps as input to a predictive network trained to determine fetal parameter (e.g., a neural network regressor, such as a convolutional neural network, large multimodal model, attention network, large language model, neural network classifier, object detection model, or segmentation network). Execution then proceeds to steps 731 and 734.

In step 731, the method 700 includes generating, as an output of the predictive network, an estimated fetal parameter for each sub-sweep. Execution then proceeds to step 732.

In step 732, the method 700 includes aggregating the fetal parameters (e.g., generating an average) from all sub-sweeps. Execution then proceeds to step 733.

Aggregation may involve generating one or more statistics based on the initial values for the fetal or anatomical parameter, including but not limited to: an average of the initial values of the fetal parameter or anatomical parameter; a maximum of the initial values of the fetal parameter or anatomical parameter; or a minimum of the initial values of the fetal parameter or anatomical parameter. The average can be a simple average or a weighted average (e.g., the fetal parameter or anatomical parameter values, weighted based on their respective confidences).

In step 733, the method 700 includes displaying the aggregated fetal parameter (e.g., the average) for all sub-sweeps.

In step 734, the method 700 includes generating, as an output of predictive network, an estimated prediction confidence for each sub-sweep. Execution then proceeds to steps 735 and 740.

In step 735, the method 700 includes aggregating confidences (e.g., averaging the confidences) from all sub-sweeps. Execution then proceeds to step 736.

Aggregation may involve generating one or more statistics based on the initial values for the confidence, including but not limited to: an average of the initial values of the confidence; a maximum of the initial values of the confidence; or a minimum of the plurality of initial values of the confidence. The average can be a simple average or a weighted average.

In step 736, the method 700 includes displaying the aggregated (e.g., averaged) confidence for all sweeps.

In step 740, the method 700 includes identifying one or multiple sub-sweeps of interest with relatively higher confidence (e.g., the highest-confidence sweep or sweeps). Execution then proceeds to step 750.

In other aspects, the method 700 can identify one or multiple sub-sweeps with relatively lower confidence (e.g., the lowest-confidence sweep or sweeps). The user may have moved the ultrasound probe too quickly for these sub-sweep(s), which resulted in the relatively lower confidence. It may not be that the sub-sweep(s) is actually a poor region to determine the fetal parameter, but rather than the data acquired from that region was poor (e.g., because the ultrasound probe was moved too quickly), which resulted in the low confidence. By recommending that the user perform a follow-up scan, there is another chance to obtain better quality data at that location (e.g., with the ultrasound probe moved at the correct speed).

In step 750, the method 700 includes generating a confidence heatmap highlighting one or multiple sub-sweeps of interest with relatively higher confidence. Execution then proceeds to steps 760 and 770.

In step 760, the method 700 includes displaying the confidence heatmap.

In step 770, the method 700 includes generating an instruction for the user to perform follow-up scan(s) with the ultrasound probe on one or multiple sub-sweeps of interest. Execution then proceeds to step 780.

In step 780, the method 700 includes displaying the instruction for the user to perform the follow-up scan (s).

Flow diagrams and block diagrams are provided herein for exemplary purposes; a person of ordinary skill in the art will recognize myriad variations that nonetheless fall within the scope of the present disclosure. For example, any of the steps described herein may optionally include an output to a user of information relevant to the step, and may thus represent an improvement in the user interface over existing art by providing information (whether static or dynamically updated) that is not otherwise available.

Similarly, block diagrams may show a particular arrangement of components, modules, services, steps, processes, or layers, resulting in a particular data flow. It is understood that some embodiments of the systems disclosed herein may include additional components, that some components shown may be absent from some embodiments, and that the arrangement of components may be different than shown, resulting in different data flows while still performing the methods described herein.

The logic of flow diagrams may be shown as sequential. However, similar logic could be parallel, massively parallel, object oriented, real-time, event-driven, cellular automaton, or otherwise, while accomplishing the same or similar functions. In order to perform the methods described herein, a processor may divide each of the steps described herein into a plurality of machine instructions, and may execute these instructions at the rate of several hundred, several thousand, several million, or several billion per second, in a single processor or across a plurality of processors. Such rapid execution may be necessary in order to execute the method in real time or near-real time as described herein. For example, real-time division of the blind sweeps into sub-sweeps, and real-time determination of the fetal parameters, may require the system to execute at speeds of 10 Hz, 30 Hz, 60 Hz, etc.

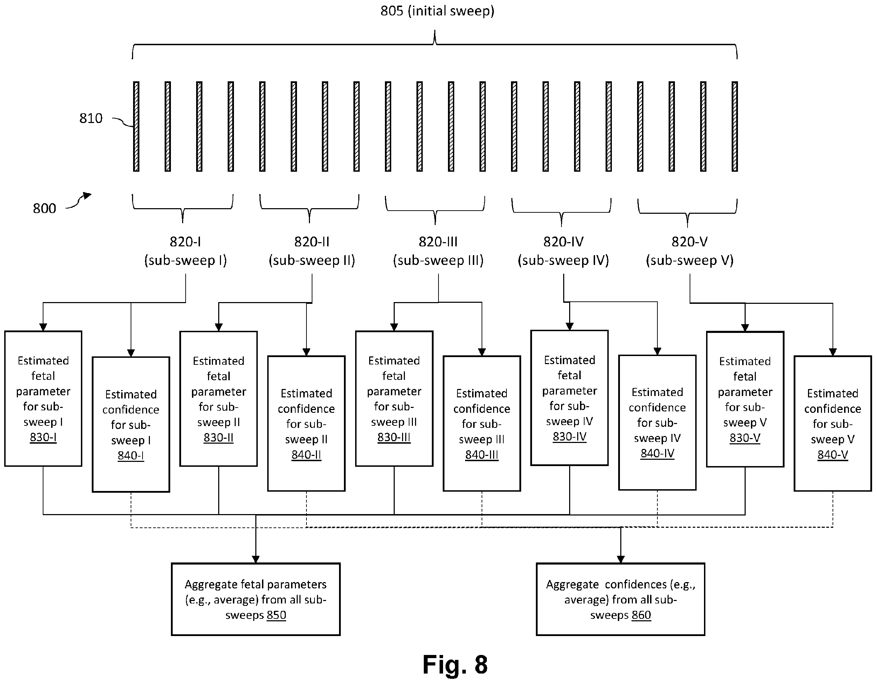

Fig. 8 is a schematic, diagrammatic representation, in block diagram form, of at least a portion of an example ultrasound sweep fetal parameter detection system 800, according to aspects of the present disclosure. In the example shown in Fig. 8, an initial sweep 805 is made up of image frames or groups of image frames 810. In an example, each rectangle 810 represents a group of 10 frames, so the 20 groups of frames 810 of Fig. 8 represent the 200 frames of the initial sweep 805.

The initial sweep 805 is then divided into five sub-sweeps 820-I through 820-V (e.g., each sub-sweep having 40 frames 810), although other numbers of sub-sweeps may be used instead or in addition. For each sub-sweep 820-I through 820-IV, an estimated fetal parameter 830 and estimated confidence 840 is computed. These estimated fetal parameters 830-I through 830-V are then aggregated (e.g., averaged) to yield an aggregated fetal parameter 850 for all sub-sweeps 820 of the sweep 805. Similarly, the estimated confidences 840-I through 840-V are aggregated (e.g., averaged) to yield an aggregated confidence 860 for all sub-sweeps 820 of the sweep 805.

Although Fig. 8 shows a single horizontal sweep 805, it is understood that the same principles can be applied to other horizontal or vertical sweeps, or any other suitable type of sweep (e.g., diagonal) in the blind sweep protocol.

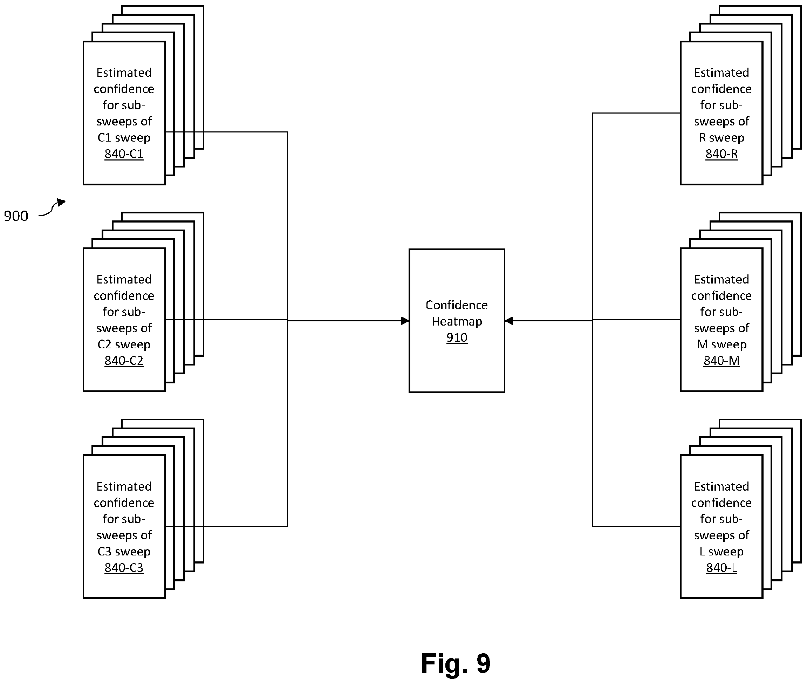

Fig. 9 is a schematic, diagrammatic representation, in block diagram form, of at least a portion of an example ultrasound sweep fetal parameter detection system 900, according to aspects of the present disclosure. In the example shown in Fig. 9, for the C1 sweep there are five estimated sub-sweep confidences 840-C1. Similarly, for the C2 sweep there are five estimated sub-sweep confidences 840-C2, for the C3 sweep there are five estimated sub-sweep confidences 840-C3, for the R sweep there are five estimated sub-sweep confidences 840-R, for the M sweep there are five estimated sub-sweep confidences 840-M, and for the L sweep there are five estimated sub-sweep confidences 840-L. Since each sub-sweep is associated with a location on the blind sweep pattern, these confidence values 840 can be combined into a confidence heatmap 910.

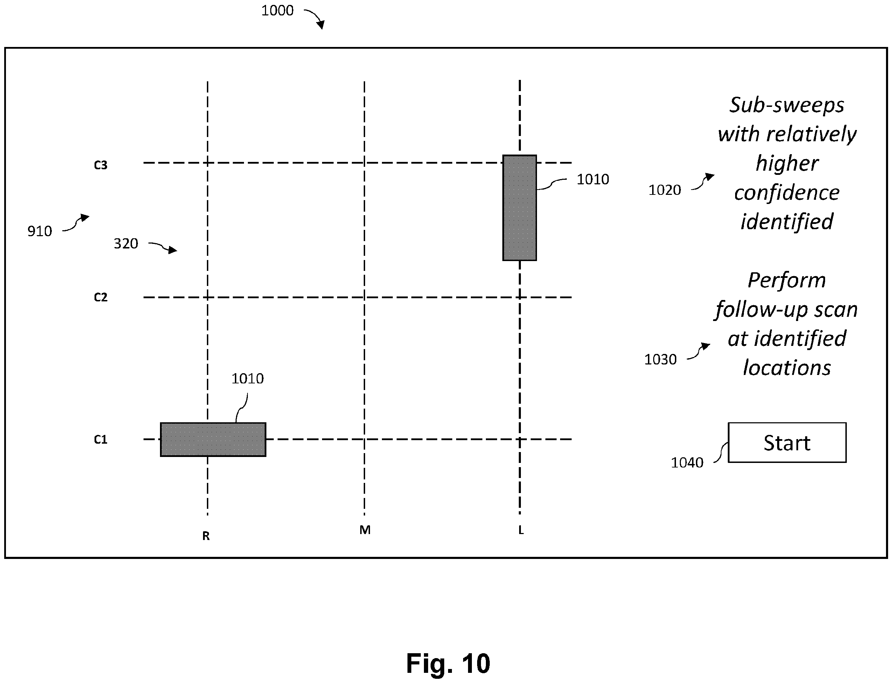

Fig. 10 is a screen display 1000 of an example ultrasound sweep fetal parameter detection system, according to aspects of the present disclosure. The screen display 1000 includes a confidence heatmap 910. The confidence heatmap 910 includes a blind sweep pattern 320 overlaid with high-confidence sub-sweeps 1010 (e.g., sub-sweeps whose confidence is relatively larger than that of the other sub-sweeps). Identification of high-confidence sub-sweeps may be done by a variety of different methods. One example is to compare the confidences from the sub-sweeps to a threshold confidence (e.g., 90% confidence) and identify the confidences/sub-sweeps that have a confidence exceeding the threshold value. Another example is to select the one, two, three, etc. highest confidences from all of the confidences of all of the sub-sweeps. Another example is to use a combination of these two methods, e.g., to pick the one, two, three, etc., highest confidences, as long as they satisfy a threshold confidence. In the example shown in Fig. 10, there are two high-confidence sub-sweeps 1010, but it is understood that there could be anywhere from one high-confidence sub-sweep 1010 (e.g., the sub-sweep with the single highest aggregate confidence 840) to several dozen high-confidence sub-sweeps 1010 (e.g., if a majority of the sub-sweeps have an aggregate confidence 840 that exceeds a threshold value).

The screen display 1000 also includes explanatory text 1020, an instruction 1030 to the user, and a start button 1040.

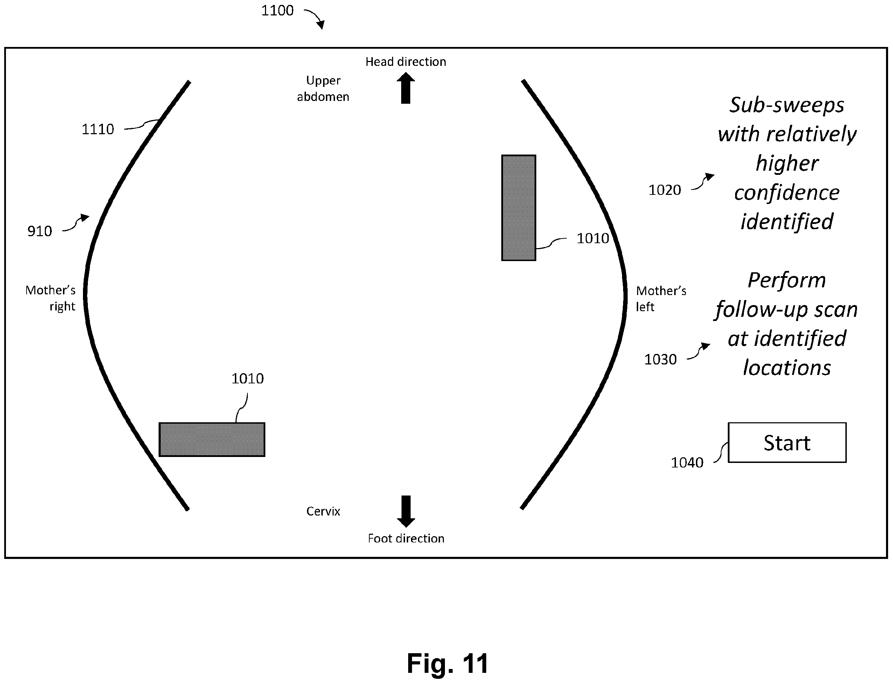

Fig. 11 is a screen display 1100 of an example ultrasound sweep fetal parameter detection system, according to aspects of the present disclosure. The screen display 1100 includes a confidence heatmap 910. The screen display 1100 is similar to the screen display 1000 of Fig. 10, except that the confidence heatmap 910 includes a representation 1110 of the patient's abdomen overlaid with high-confidence sub-sweeps 1010. The screen display 1000 also includes explanatory text 1020, an instruction 1030 to the user, and a start button 1040.

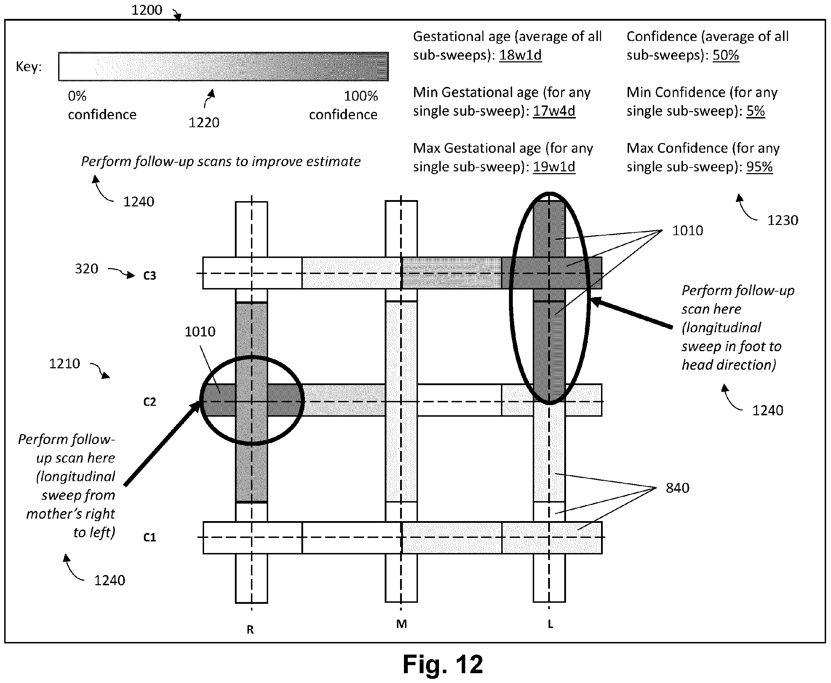

Fig. 12 is a screen display 1200 of an example ultrasound sweep fetal parameter detection system, according to aspects of the present disclosure. The screen display includes a confidence heatmap 1210 overlaid on a blind sweep pattern 320. Unlike the heatmap 910 of Figures 10 and 11, the heatmap 1210 of Fig. 12 shows all of the sub-sweeps 840. However, the sub-sweeps 840 are color-coded according to a key 1220, such that (for example) the highest-confidence sweeps. The screen display 1200 also includes explanatory text and statistics 1230, which may for example include the maximum, minimum, and average values for the fetal parameter, as well as the maximum, minimum, and average values for the prediction confidence. The screen display 1200 also includes user instructions 1240, which may for example direct the user to perform follow-up scans in the locations of the highest-confidence sub-sweeps 1010. The follow-up scan can be vertical movement or horizontal movement of the ultrasound probe (e.g., matching the corresponding sub-sweep), or any other suitable movement for capturing high-quality ultrasound images of the region of interest, depending on the type of fetal parameter being measured. Depending on the implementation, the user instructions 1240 may include information about how the user should move the ultrasound probe.

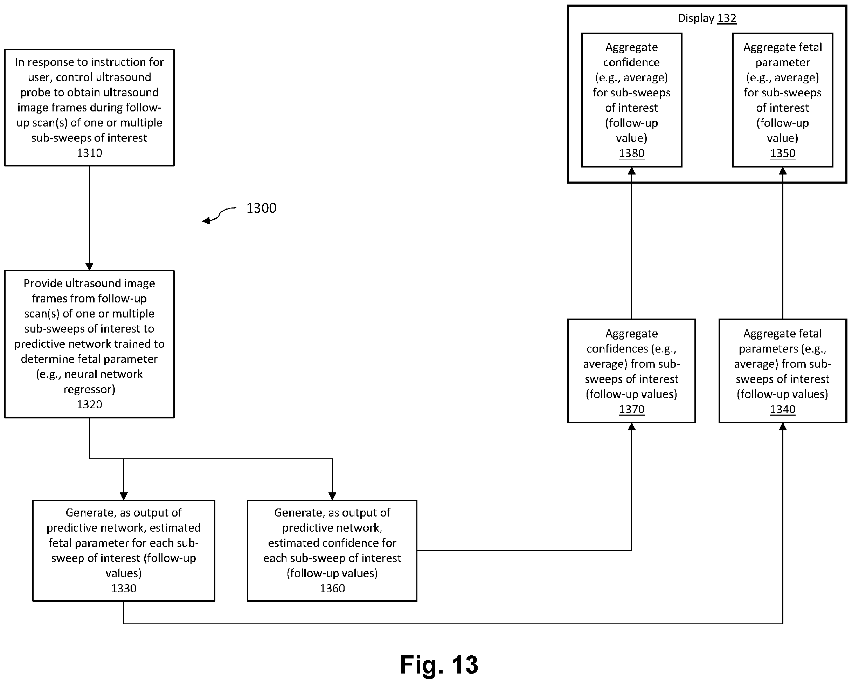

Fig. 13 is a schematic, diagrammatic representation, in flow diagram form, of an example ultrasound sweep fetal parameter detection method 1300, according to aspects of the present disclosure. The method 1300 may for example be performed after the user has completed the initial blind sweep protocol.

In step 1310, the method 1300 includes, in response to instruction given to the user, controlling ultrasound probe to obtain ultrasound image frames during follow-up scan(s) of one or multiple sub-sweeps of interest. Execution then proceeds to step 1320.

In step 1320, the method 1300 includes providing the ultrasound image frames, from the follow-up scan(s) of the one or multiple sub-sweeps of interest, to a predictive network trained to determine fetal parameter (e.g., a neural network regressor). Execution then proceeds to steps 1330 and 1360.

In step 1330, the method 1300 includes generating, as output of the predictive network, an estimated fetal parameter for each sub-sweep of interest (e.g., the follow-up values for the fetal parameter).

In step 1340, the method 1300 includes aggregating the fetal parameters from the sub-sweeps of interest (e.g., averaging the follow-up values of the fetal parameter). Execution then proceeds to step 1350.

In step 1350, the method 1300 includes displaying the aggregate fetal parameter (e.g., the average of the follow-up values for the fetal parameter) on the display 132.

In step 1360, the method 1300 includes generating, as an output of the predictive network, an estimated confidence for each sub-sweep of interest (e.g., the follow-up values for fetal parameter prediction confidence). Execution then proceeds to step 1370.

In step 1370, the method 1300 includes aggregating the fetal parameter prediction confidences from the sub-sweeps of interest (e.g., averaging the confidence values). Execution then proceeds to step 1380.

In step 1380, the method 1300 includes displaying the aggregate confidence (e.g., the average of the follow-up values for the fetal parameter prediction confidence) on the display 132.

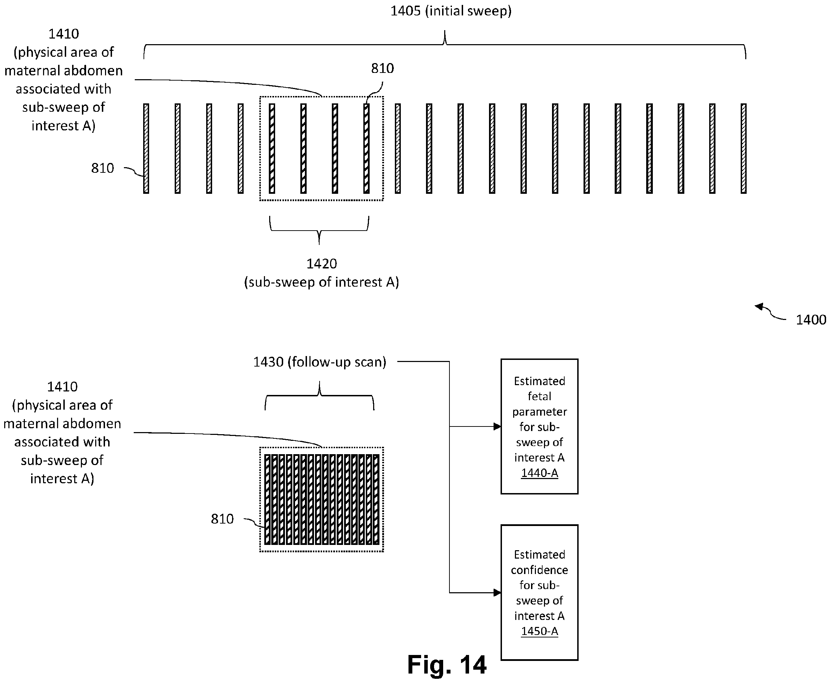

Fig. 14 is a schematic, diagrammatic representation of a follow-up scanning process 1400, according to aspects of the present disclosure. An initial sweep 1405 is made up of ultrasound image frames or groups of image frames 810. The initial sweep 1405 includes a sub-sweep of interest A, 1420 (e.g., a sub-sweep with high fetal parameter prediction confidence), which takes place in a physical area of interest A, 1410. A follow-up scan 1430 in the same physical area 1410 (associated with the sub-sweep of interest A) yields an estimated fetal parameter 1440-A and an estimated confidence value 1450-A for the follow-up scan of sub-sweep of interest A. It is noted that the follow-up scan 1430 may include substantially more image frames 810 than the initial sub-sweep of interest A, 1420. For example, the sub-sweep 1420 could have 30, 40, 50 frames (and/or other suitable values both larger and smaller), whereas the follow up scan 1430 could have 175, 200, 225 frames (and/or other suitable values both larger and smaller). This may provide greater accuracy and confidence in the prediction of the fetal parameter, by providing greater resolution for the fetal and/or maternal anatomy in the physical area of interest A, 1410. It is understood that there may be multiple sub-sweeps of interest, each resulting in a follow-up scan and an estimated fetal parameter and confidence value, as described below.

The follow-up value of the fetal parameter and/or the follow-up value of the confidence is thus determined using a greater quantity of ultrasound image frames 810 than used to determine the initial value of the fetal parameter and/or the confidence. This can be described as a greater resolution or greater density of ultrasound image frames 810, because the same physical region of interest is being imaged with a greater number of ultrasound image frames in the follow-up scan compared to the number of ultrasound image frames in the sub-sweep of interest.

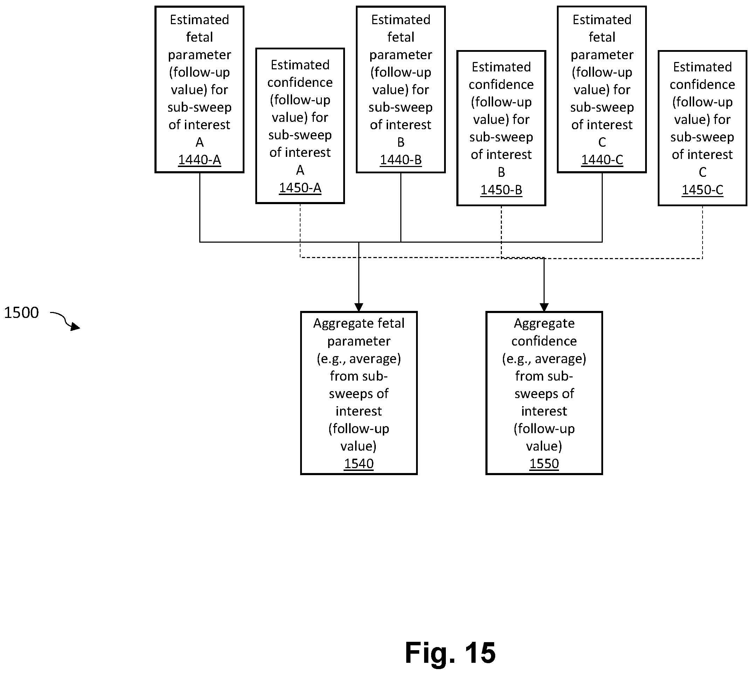

Fig. 15 is a schematic, diagrammatic representation, in block diagram form, of at least a portion of an example ultrasound sweep fetal parameter detection system 1500, according to aspects of the present disclosure. In the example shown in Fig. 15, three different sub-sweeps of interest - sub-sweep A, sub-sweep B, and sub-sweep C - have respectively generated a follow-up estimated fetal parameter 1440-A and follow-up estimated confidence 1450-A, follow-up estimated fetal parameter 1440-B and follow-up estimated confidence 1450-B, and follow-up estimated fetal parameter 1440-C and follow-up estimated confidence 1450-C. The fetal parameter values 1440-A, 1440-B, and 1440-C can then be averaged or otherwise aggregated to form an aggregate follow-up fetal parameter 1540 from the sub-sweeps of interest. Similarly, the confidence values 1450-A, 1450-B, and 1450-C can be averaged or otherwise aggregated to yield an aggregate follow-up confidence 1550 for the sub-sweeps of interest. One object of the present disclosure is that the accuracy and confidence of these aggregate values 1540, 1550 be higher than the values 733, 736 (see Fig. 7) obtained from the original high-confidence sub-sweeps 1010 (see Fig. 10).

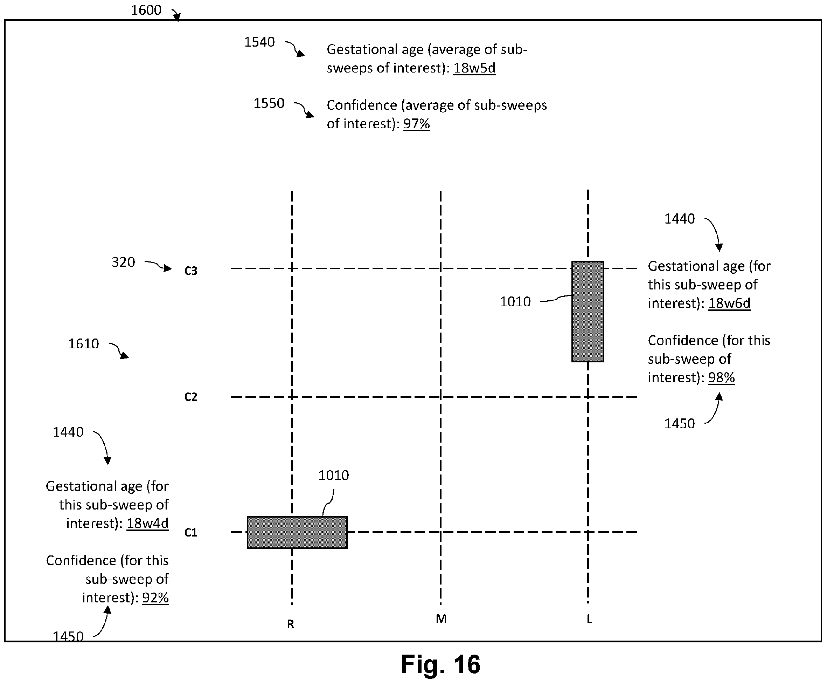

Fig. 16 is screen display 1600 for an example ultrasound sweep fetal parameter detection system, according to aspects of the present disclosure. The screen display 1600 includes a confidence heatmap 1610 overlaid on a blind sweep pattern 320. The confidence heatmap 1610 includes the highest-confidence sub-sweeps 1010 (e.g., sub-sweeps for which the confidence exceeds a threshold value). The screen display 1600 is similar in some regards to the screen display 1000 of Fig. 10. However, unlike the screen display 1000 of Fig. 10, the screen display 1600 includes a follow-up estimated fetal parameter 1440 and follow-up estimated confidence 1450 for each sub-sweep of interest 1010. The screen display 1600 also includes an aggregated follow-up fetal parameter 1540 and an aggregated follow-up confidence 1550, as described above in Fig. 15. Thus, the screen display 1600 allows the user to see, at a glance, the results of re-scanning the sub-sweeps of interest, and to receive a more accurate, more confident estimate of the fetal parameter (e.g., 97 % confidence for the follow-up aggregated gestational age, vs. 50% confidence for the initial aggregated gestational age).

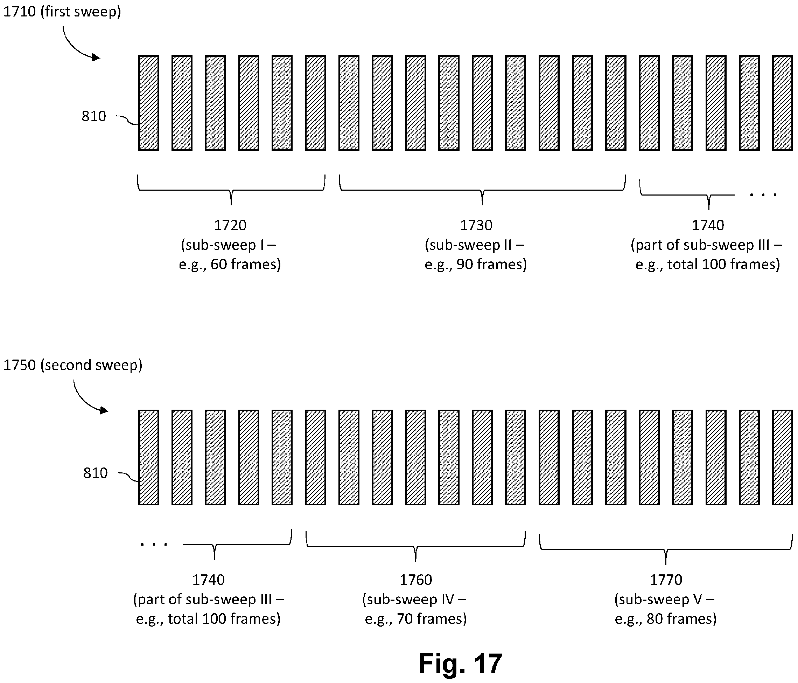

Fig. 17 is a schematic, diagrammatic representation of the division of sweeps into sub-sweeps of variable size, according to aspects of the present disclosure. This may be done for example to ensure that each sub-sweep contains only a single identified maternal or fetal anatomy, or that each sub-sweep contains multiple anatomies. In the example shown in Fig. 17, a first horizontal sweep 1710, composed of image frames 810, is divided into two complete sub-sweeps 1720 and 1730, and one incomplete sub-sweep 1740 that is shared with a second horizontal sweep 1750. Similarly, the second horizontal sweep 1750 is divided into one incomplete sub-sweep 1740 (shared with the first horizontal sweep 1710) and two complete sub-sweeps 1760 and 1770. Each of these sub-sweeps has a different length; sub-sweep 1720 includes 60 ultrasound image frames, whereas sub-sweep 1730 includes 90 frames, sub-sweep 1740 includes a total of 100 frames (e.g., 50 from sweep 1710 and 50 from sweep 1750), sub-sweep 1760 includes 70 frames, and sub-sweep 1770 includes 80 frames. These values are merely exemplary. Depending on the implementation, the actual length of a sub-sweep could be as small as one frame or as large as several hundred frames. Although Fig. 17 shows two horizontal sweeps 1710, 1750m the same process can be performed with two vertical sweeps, one horizontal sweep and one vertical sweep, etc.

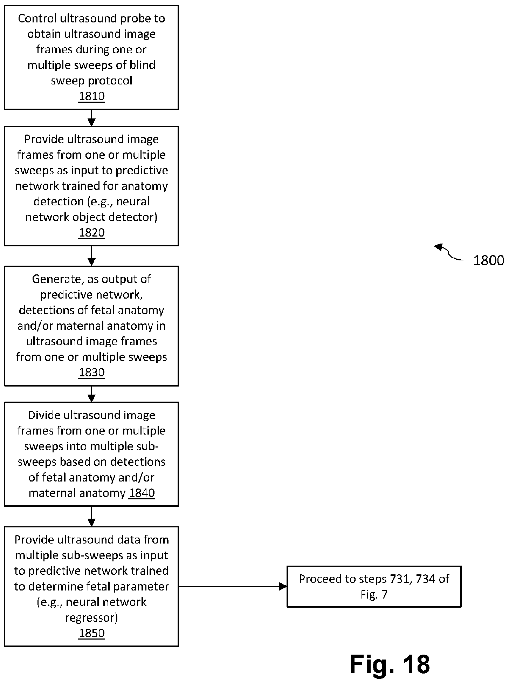

Fig. 18 is a schematic, diagrammatic representation, in flow diagram form, of an example ultrasound sweep fetal parameter detection method 1800, according to aspects of the present disclosure.

In step 1810, the method 1800 includes controlling the ultrasound probe to obtain ultrasound image frames during one or multiple sweeps of a blind sweep protocol. Execution then proceeds to step 1820.

In step 1820, the method 1800 includes providing the ultrasound image frames from the one or multiple sweeps as input to predictive network trained for anatomy detection (e.g., a neural network object detector, such as a convolutional neural network, YOLO, perceptron, RCNN, or Mobilenet). Execution then proceeds to step 1830. The neural network object detector may be different than and/or separate from the neural network regressor (e.g., they could be separate neural networks).

In step 1830, the method 1800 includes generating, as an output of the predictive network, detections of fetal anatomy and/or maternal anatomy in the ultrasound image frames from the one or multiple sweeps. Execution then proceeds to step 1840.

In step 1840, the method 1800 includes dividing the ultrasound image frames from the one or multiple sweeps into multiple sub-sweeps based on detections of fetal anatomy and/or maternal anatomy. Execution then proceeds to step 1850.

In step 1850, the method 1800 includes providing the ultrasound data from the multiple sub-sweeps as input to a predictive network trained to determine a fetal parameter (e.g., a neural network regressor). Execution then proceeds to steps 731 and 734 of Fig. 7.

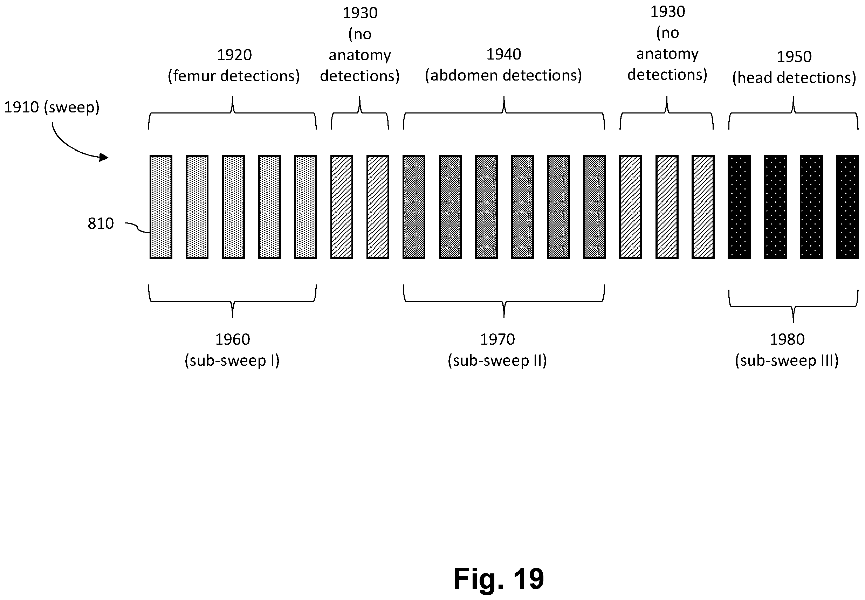

Fig. 19 is a schematic, diagrammatic representation of the division of sweeps into sub-sweeps of variable size, according to aspects of the present disclosure. In the example shown in Fig. 19, image frames in which no anatomy was detected may be excluded altogether from the sub-sweeps. For example, in a sweep 1910, some frames 810 include fetal femur detections, some frames 1930 contain no anatomy detections, some frames 1940 include fetal abdomen detections, and some frames 810 include head detections 1950. A first sub-sweep 1960 (sub-sweep I) includes only those frames 810 with femur detections 1920. A second sub-sweep 1970 (sub-sweep II) includes only those frames 810 with abdomen detections 1940. A third sub-sweep 1980 (sub-sweep III) includes only those frames 810 with head detections 1950. This division of frames into sub-sweeps may result in generally higher accuracy and confidence for fetal parameter prediction, by excluding frames that do not include fetal anatomy, and by constructing sub-sweeps that focus on a particular anatomy that is useful for predicting a fetal parameter such as gestational age.

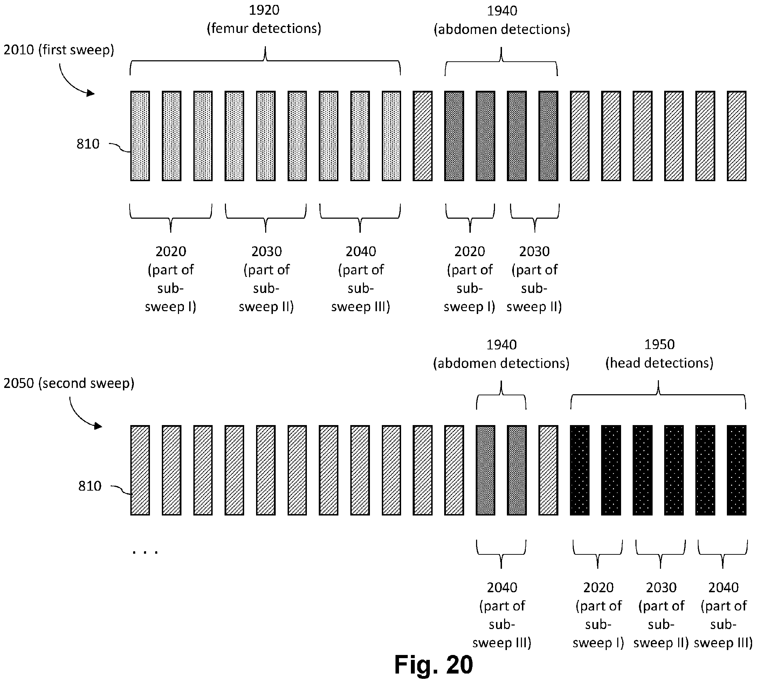

Fig. 20 is a schematic, diagrammatic representation of the division of sweeps into sub-sweeps of variable size, according to aspects of the present disclosure. In the example shown in Fig. 19, image frames in which no anatomy was detected may be excluded altogether from the sub-sweeps, and sub-sweeps may be constructed from discontinuous frames containing multiple anatomies of interest. A first sweep 2010 includes image frames 810 containing femur detections 1920 and abdomen detections 1940. A second sweep 2050 incudes image frames 810 containing abdomen detections 1940 and head detections 1950. Parts 2020 of a first sub-sweep (sub-sweep I) are distributed across the two sweeps 2010, 2050 such that some of the parts 2020 include femur detections 1920, some of the parts 2020 include abdomen detections 1940, and some parts 2020 include head detections 1950. Thus, sub-sweep I includes detections of all three anatomies 1920, 1940, 1950. Similarly, parts 2030 of a second sub-sweep (sub-sweep II) are distributed across the two sweeps 2010, 2050 such that some of the parts 2030 include femur detections 1920, some of the parts 2030 include abdomen detections 1940, and some parts 2030 include head detections 1950. Parts 2040 of a third sub-sweep (sub-sweep III) are distributed across the two sweeps 2010, 2050 such that some of the parts 2040 include femur detections 1920, some of the parts 2040 include abdomen detections 1940, and some parts 2040 include head detections 1950. Thus, each of sub-sweeps I, II, and III includes frames 810 containing detections of all three anatomies 1920, 1940, 1950. This division of frames into sub-sweeps may result in generally higher accuracy and confidence for fetal parameter prediction, by excluding frames that do not include fetal anatomy, and by constructing sub-sweeps that each include multiple anatomies that are useful for predicting a fetal parameter such as gestational age.

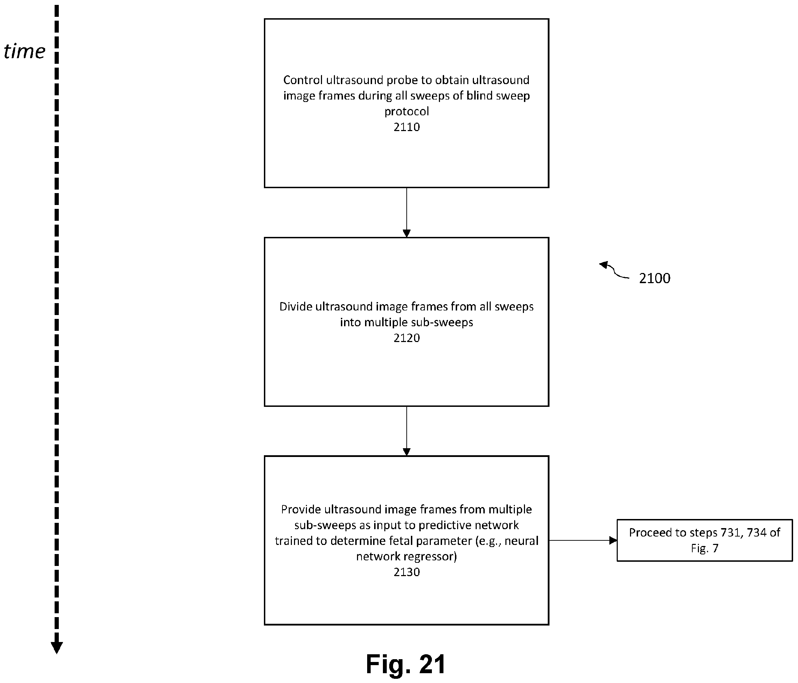

Fig. 21 is a schematic, diagrammatic representation, in flow diagram form, of an example ultrasound sweep fetal parameter detection method 2100, according to aspects of the present disclosure.

In step 2110, the method 2100 includes controlling the ultrasound probe to obtain ultrasound image frames during all sweeps of the blind sweep protocol. Execution then proceeds to step 2120.

In step 2120, the method 2100 includes dividing the ultrasound image frames from all sweeps into multiple sub-sweeps, for example as described above in Figs. 17, 19, and 20. Execution then proceeds to step 2130.

In step 2130, the method 2100 includes provide the ultrasound image frames from multiple sub-sweeps as input to a predictive network trained to determine a fetal parameter (e.g., a neural network regressor). Execution then proceeds to steps 731 and 724 of Fig. 7.

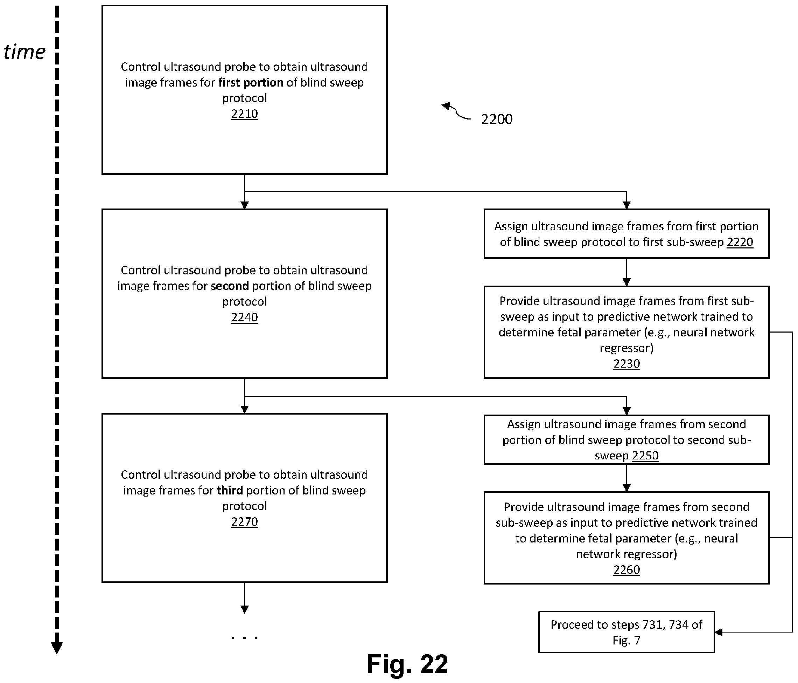

Fig. 22 is a schematic, diagrammatic representation, in flow diagram form, of an example real-time or near-real time ultrasound sweep fetal parameter detection method 2200, according to aspects of the present disclosure. The method 2200 is similar to the method 2100 of Fig. 1, except that the capture of ultrasound image frames from the blind sweep protocol is divided into portions. These portions may each be complete sweeps, incomplete sweeps, or may comprise multiple sweeps or portions thereof.

In step 2210, the method 2200 includes controlling the ultrasound probe to obtain image frames for a first portion of the blind sweep protocol (e.g., 30, 40, 50 frames, and/or other values both larger and smaller). Execution then proceeds to steps 2220 and 2240.

In step 2220, the method 2200 includes assigning ultrasound image frames from the first portion of the blind sweep protocol to the first sub-sweep. Execution then proceeds to step 2230.

In step 2230, the method 2200 includes providing the ultrasound image frames from the first sub-sweep as input to a predictive network trained to determine a fetal parameter (e.g., a neural network regressor). Execution then proceeds to steps 731 and 734 of Fig. 7.

In step 2240, the method 2200 includes controlling the ultrasound probe to obtain image frames for a second portion of the blind sweep protocol (e.g., the next 30, 40, 50 frames, and/or other values both larger and smaller). Execution then proceeds to steps 2250 and 2270.

In step 2250, the method 2200 includes assigning ultrasound image frames from the first portion of the blind sweep protocol to the first sub-sweep. Execution then proceeds to step 2260.

In step 2260, the method 2200 includes providing the ultrasound image frames from the second sub-sweep as input to a predictive network trained to determine a fetal parameter (e.g., a neural network regressor). Execution then proceeds to steps 731 and 734 of Fig. 7.

In step 2270 the method 2200 includes controlling the ultrasound probe to obtain image frames for a third portion of the blind sweep protocol (e.g., the next 30, 40, 50 frames, and/or other values both larger and smaller). Execution then proceeds to additional steps as needed, depending on the number of portions of the blind sweep protocol that will be processed. These additional steps may for example be comparable to steps 2250 and 2270.

As will be readily appreciated by those having ordinary skill in the art after becoming familiar with the teachings herein, the ultrasound sweep fetal parameter detection system advantageously permits untrained and minimally trained users to perform an ultrasound blind sweep protocol to gather anatomical images and measurements of high quality, including automated detection of fetal parameters such as gestational age. This may result in higher accuracy and higher clinician trust in the results, while potentially improving health outcomes and/or decreasing the total cost of care.

The systems, methods, and devices described herein may be applicable in point of care and handheld ultrasound use cases such as with the Philips Lumify system. The ultrasound sweep fetal parameter detection system can be used for any handheld imaging applications, including but not limited to obstetrics, lung imaging, and echocardiography. The ultrasound sweep fetal parameter detection system could be deployed on handheld mobile ultrasound devices, and on portable or cart-based ultrasound systems. The ultrasound sweep fetal parameter detection system can be used in a variety of settings including emergency departments, ambulances, accident sites, and homes. The applications could also be expanded to other settings.

The ultrasound sweep fetal parameter detection system may be detectable via visual inspection by a non-technical user, based on a workflow and user interface directed toward targeted sweep triggering, and/or a confidence heatmap of the sweeps, and/or a user interface displaying a prompt to do a follow up scan on some part of some sweep that showed higher confidence. The present disclosure increases the value proposition of ultrasound applications in the obstetrics context, especially for use by minimally trained users.

A number of variations are possible on the examples and aspects described above. For example, the systems, methods, and devices described herein are not limited to obstetric ultrasound applications. Rather, the same technology can be applied to images of other organs or anatomical systems such as the lungs, heart, brain, digestive system, vascular system, etc., for both male and female patients, with or without pregnancy, for measurement of any anatomical parameter (e.g., not just fetal parameters), whether or not related to a pregnancy. Furthermore, the technology disclosed herein is also applicable to other medical imaging modalities where 3D data are available, such as other ultrasound applications, camera-based videos, X-ray videos, and 3D volume images, such as computer aided tomography (CT) scans, magnetic resonance imaging (MRI) scans, optical coherence tomography (OCT) scans, or intravascular ultrasound (IVUS) pullback sequences. The technology described herein can be used in a variety of settings.

The logical operations making up the aspects of the technology described herein are referred to variously as operations, steps, objects, layers, elements, components, algorithms, or modules. Furthermore, it should be understood that these may occur or be performed or arranged in any order, unless explicitly claimed otherwise or a specific order is inherently necessitated by the claim language.

All directional references e.g., upper, lower, inner, outer, upward, downward, left, right, lateral, front, back, top, bottom, above, below, vertical, horizontal, clockwise, counterclockwise, proximal, and distal are only used for identification purposes to aid the reader's understanding of the claimed subject matter, and do not create limitations, particularly as to the position, orientation, or use of the ultrasound sweep fetal parameter detection system. Connection references, e.g., attached, coupled, connected, joined, or "in communication with" are to be construed broadly and may include intermediate members between a collection of elements and relative movement between elements unless otherwise indicated. As such, connection references do not necessarily imply that two elements are directly connected and in fixed relation to each other. The term "or" shall be interpreted to mean "and/or" rather than "exclusive or." The word "comprising" does not exclude other elements or steps, and the indefinite article "a" or "an" does not exclude a plurality. Unless otherwise noted in the claims, stated values shall be interpreted as illustrative only and shall not be taken to be limiting.

The above specification, examples and data provide a complete description of the structure and use of exemplary aspects of the ultrasound sweep fetal parameter detection system as defined in the claims. Although various aspects of the claimed subject matter have been described above with a certain degree of particularity, or with reference to one or more individual aspects, those skilled in the art could make numerous alterations to the disclosed aspects without departing from the spirit or scope of the claimed subject matter.

Still other aspects are contemplated. It is intended that all matter contained in the above description and shown in the accompanying drawings shall be interpreted as illustrative only of particular aspects and not limiting. Changes in detail or structure may be made without departing from the basic elements of the subject matter as defined in the following claims.

## Claims

1. An apparatus, comprising:
a processor circuit configured for communication with an ultrasound probe, wherein the processor circuit is configured to:
control the ultrasound probe to obtain a first plurality of ultrasound image frames during one or more sweeps of a blind sweep protocol on a patient with a pregnancy;
divide the first plurality of ultrasound image frames into a plurality of sub-sweeps;
determine, based on the first plurality of ultrasound image frames, a plurality of initial values of a fetal parameter and a plurality of initial values of a confidence for the plurality of sub-sweeps, respectively;
determine an initial value of the confidence that is relatively larger than other initial values of the confidence, wherein the initial value of the confidence that is identified is specific to a sub-sweep of the plurality of sub-sweeps; and
output, to a display in communication with the processor circuit, a first screen display comprising an instruction to a user to perform a follow-up scan with the ultrasound probe on the sub-sweep associated to the identified initial value of confidence.

2. The apparatus of claim 1,
wherein the processor circuit is configured to generate a heatmap based on the plurality of initial values of the confidence,
wherein the first screen display comprises the heatmap.

3. The apparatus of claim 2, wherein the heatmap identifies a location of the sub-sweep.

4. The apparatus of claim 2 or 3, wherein the plurality of sub-sweeps is respectively color-coded in the heatmap based on the plurality of initial values of the confidence.

5. The apparatus of any of claims 2-4, wherein the heatmap comprises a graphical representation of the one or more sweeps of the blind sweep protocol and/or a graphical presentation of an abdomen of the patient.

6. The apparatus of any of claims 1-5,
wherein the processor circuit is configured to generate one or more statistics based on at least one of:
the plurality of initial values of the fetal parameter; or
the plurality of initial values of the confidence,
wherein the first screen display comprises the one or more statistics.

7. The apparatus of any of claims 1-6, wherein a quantity of the first plurality of ultrasound image frames in each sub-sweep of the plurality of sub-sweeps is less than a quantity of the first plurality of ultrasound image frames in a single sweep of the plurality of sweeps.

8. The apparatus of any of claims 1-7,
wherein the processor circuit is further configured to detect one or more fetal anatomies in the first plurality of ultrasound images frames,
wherein the first plurality of ultrasound image frames are divided into the plurality of sub-sweeps based on the one or more fetal anatomies, and
optionally wherein, to detect the one or more fetal one or more fetal anatomies, the processor circuit is configured to:
provide the first plurality of ultrasound image frames as an input to an object detection neural network trained to detect the one or more fetal anatomies; and
detect the one or more fetal anatomies as an output of the object detection neural network.

9. The apparatus of any of claims 1-8, wherein the processor circuit is configured to control the ultrasound probe to obtain a second portion of the first plurality of ultrasound image frames simultaneously as at least one:
the processor circuit assigning a first portion of the first plurality of ultrasound image frames to a given sub-sweep; or
the processor circuit determining, using the first portion of the first plurality of ultrasound image frames, a given initial value of the fetal parameter and a given initial value of the confidence for the given sub-sweep.

10. The apparatus of any of claims 1-9, wherein the processor circuit is configured to:
control the ultrasound probe to obtain a second plurality of ultrasound image frames during the follow-up scan on the sub-sweep;
determine, based on the second plurality of ultrasound image frames, a follow-up value of the fetal parameter and a follow-up value of the confidence for the follow-up scan; and
output, to the display, a second screen display comprising at least one of:
the follow-up value of the fetal parameter; or
the follow-up value of the confidence, and
optionally wherein a quantity of the second plurality of ultrasound image frames in the follow-up scan is greater than the quantity of the first plurality of ultrasound image frames in the sub-sweep.

11. The apparatus of claim 10,
wherein the processor circuit is configured to determine a further initial value of the confidence that is relatively larger than the other initial values of the confidence, wherein the further initial value of the confidence that is identified is specific to a further sub-sweep of the plurality of sub-sweeps;
wherein the instruction to the user is to perform a further follow-up scan with the ultrasound probe on the further sub-sweep.

12. The apparatus of claim 11,
wherein the second plurality of ultrasound image frames comprises the further follow-up scan on the further sub-sweep,
wherein the processor circuit is configured to:
determine, based on the second plurality of ultrasound image frames, a further follow-up value of the fetal parameter and a further follow-up value of the confidence for the further follow-up scan; and
generate one or more statistics based on at least one of:
the follow-up value of the fetal parameter for the follow-up scan and the further follow-up value of the fetal parameter for the further follow-up scan; or
the follow-up value of the confidence for the follow-up scan and the further follow-up value of the confidence for the further follow-up scan, and
wherein the second screen display comprises the one or more statistics.

13. The apparatus of any of claims 1-12, wherein the processor circuit is configured to:
provide the first plurality of ultrasound image frames as an input to a regression neural network trained to determine the plurality of initial values of the fetal parameter and the plurality of initial values of the confidence; and
determine the plurality of initial values of the fetal parameter and the plurality of initial values of the confidence as an output of the regression neural network.

14. A method for providing guidance to a user to perform a follow-up scan to determine a value of a fetal parameter using ultrasound data from a blind abdominal imaging sweep, comprising:
controlling an ultrasound probe to obtain a first plurality of ultrasound image frames during one or more sweeps of a blind sweep protocol on a patient with a pregnancy;
dividing the first plurality of ultrasound image frames into a plurality of sub-sweeps;
determining, based on the first plurality of ultrasound image frames, a plurality of initial values of a fetal parameter and a plurality of initial values of a confidence for the plurality of sub-sweeps, respectively;
determining an initial value of the confidence that is relatively larger than other initial values of the confidence, wherein the initial value of the confidence that is identified is specific to a sub-sweep of the plurality of sub-sweeps; and
outputting, to a display, a first screen display comprising an instruction to a user to perform a follow-up scan with the ultrasound probe on the sub-sweep associated to the identified initial value of confidence.

15. A non-transitory computer-readable medium storing instructions that, when executed by a processor, cause the processor to perform the method of claim 14.
